(19) Europäisches Patentamt / European Patent Office / Office européen des brevets

(11) EP 2 333 115 A1

(12) **EUROPEAN PATENT APPLICATION**

(43) Date of publication:
**15.06.2011 Bulletin 2011/24**

(51) Int Cl.:
***C12Q 1/68*** *(2006.01)* ***C12N 15/10*** *(2006.01)*

(21) Application number: **10187058.2**

(22) Date of filing: **09.05.2006**

(84) Designated Contracting States:
**AT BE BG CH CY CZ DE DK EE ES FI FR GB GR HU IE IS IT LI LT LU LV MC NL PL PT RO SE SI SK TR**

(30) Priority: **22.12.2005 PCT/NL2005/000884**

(62) Document number(s) of the earlier application(s) in accordance with Art. 76 EPC:
**06733048.0 / 1 974 053**

(71) Applicant: **Keygene N.V.**
**6700 AE Wageningen (NL)**

(72) Inventors:
- **Bundock, Paul**
  **6700 AE Wageningen (NL)**
- **de Both, Michiel Theodoor Jan**
  **6700 AE Wageningen (NL)**
- **Hogers, René Cornelis Josephus**
  **6700 AE Wageningen (NL)**

(74) Representative: **Raggers, René John**
**Exter Polak & Charlouis B.V.**
**P.O. Box 3241**
**2280 GE Rijswijk (NL)**

Remarks:
This application was filed on 08-10-2010 as a divisional application to the application mentioned under INID code 62.

# (54) Alternative nucleotides for improved targeted nucleotide exchange

(57) A method and oligonucleotides for targeted nucleotide exchange of a duplex DNA sequence, wherein the donor oligonucleotide contains at least one modified nucleotide having a higher binding affinity compared to naturally occurring A, C, T or G and/or binds stronger to a nucleotide in an opposite position in the first DNA sequence as compared to a naturally occurring nucleotide complementary to the nucleotide in the opposite position in the first DNA sequence.

Fig 1

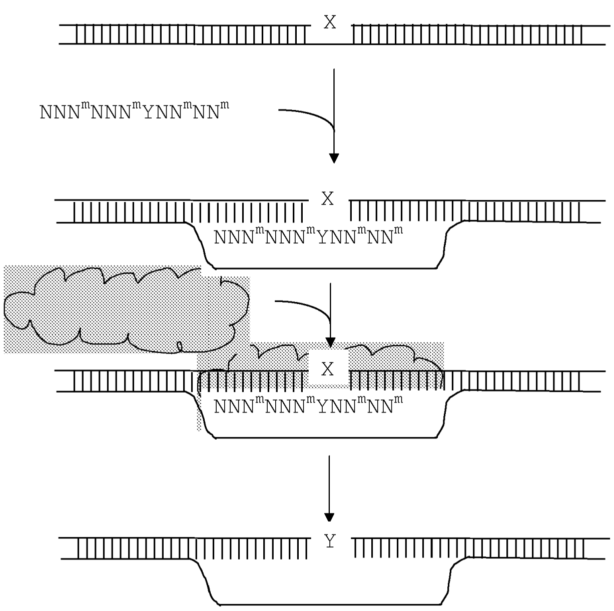


EP 2 333 115 A1

## Description

Field of the invention

The present invention relates to a method for the specific and selective alteration of a nucleotide sequence at a specific site of the DNA in a target cell by the introduction into that cell of an oligonucleotide. The result is the targeted exchange of one or more nucleotides so that the sequence of the target DNA is converted to that of the oligonucleotide where they are different. More in particular, the invention relates to the targeted nucleotide exchange using modified oligonucleotides. The invention further relates to oligonucleotides and kits. The invention also relates to the application of the method.

Background of the invention

Genetic modification is the process of deliberately creating changes in the genetic material of living cells with the purpose of modifying one or more genetically encoded biological properties of that cell, or of the organism of which the cell forms part or into which it can regenerate. These changes can take the form of deletion of parts of the genetic material, addition of exogenous genetic material, or changes in the existing nucleotide sequence of the genetic material. Methods for the genetic modification of eukaryotic organisms have been known for over 20 years, and have found widespread application in plant, human and animal cells and micro-organisms for improvements in the fields of agriculture, human health, food quality and environmental protection. The common methods of genetic modification consist of adding exogenous DNA fragments to the genome of a cell, which will then confer a new property to that cell or its organism over and above the properties encoded by already existing genes (including applications in which the expression of existing genes will thereby be suppressed). Although many such examples are effective in obtaining the desired properties, these methods are nevertheless not very precise, because there is no control over the genomic positions in which the exogenous DNA fragments are inserted (and hence over the ultimate levels of expression), and because the desired effect will have to manifest itself over the natural properties encoded by the original and well-balanced genome. On the contrary, methods of genetic modification that will result in the addition, deletion or conversion of nucleotides in predefined genomic loci will allow the precise modification of existing genes.

Currently, two methods are known for creating precise targeted genetic changes in eukaryotic cells: gene targeting through homologous recombination and oligonucleotide-directed targeted nucleotide exchange.

Methods based on homologous recombination exploit the principle that naturally occurring or pre-induced double-strand breaks in the genomic DNA will be repaired by the cell using any available template DNA fragment with some nucleotide sequence homology to the flanking regions adjacent to the break (Puchta, Plant Mol.Biol. 48: 173, 2002; J. Exp. Botany, 2005, 56, 1). By supplying to such cell donor DNA with the required sequence homologies, homologous recombination at either end of the break may result in a precise repair, whereby any artificially designed modifications in between the homology regions of the donor DNA will be incorporated in the existing loci. In eukaryotic cells, this precise break repair occurs at rather low frequencies in favour of a less precise repair mechanism, in which parts of the sequence may be lost and non-related DNA sequence may be incorporated.

Oligonucleotide-directed Targeted Nucleotide Exchange (TNE, sometimes ODTNE) is a different method, that is based on the delivery into the eukaryotic cell nucleus of synthetic oligonucleotides (molecules consisting of short stretches of nucleotide-like moieties that resemble DNA in their Watson-Crick basepairing properties, but may be chemically different from DNA) (Alexeev and Yoon, Nature Biotechnol. 16: 1343, 1998; Rice, Nature Biotechnol. 19: 321, 2001; Kmiec, J. Clin. Invest. 112: 632, 2003). By deliberately designing a mismatch nucleotide in the homology sequence of the oligonucleotide, the mismatch nucleotide may be incorporated in the genomic DNA sequence. This method allows the conversion of single or at most a few nucleotides in existing loci, but may be applied to create stop codons in existing genes, resulting in a disruption of their function, or to create codon changes, resulting in genes encoding proteins with altered amino acid composition (protein engineering).

Targeted nucleotide exchange has been described in plant, animal and yeast cells. The first TNE reports utilized a so-called chimera wherein the oligonucleotides may be synthesized as RNA-DNA hybrid molecules (Beetham et al., PNAS 96: 8774, 1999; Kochevenko and Willmitzer, Plant Physiol. 132: 174, 2003) and that consisted of a self-complementary oligonucleotide that is designed to intercalate at the chromosomal target site. The chimera contains a mismatched nucleotide that forms the template for introducing the mutation at the chromosomal target. The first examples using chimeras came from human cells (see the review Rice et al. Nat.Biotech., 2001, 19: 321-326,; Alexeev et al. Nature Biotech, 2000, 18, 43). The use of chimeras has also been successful in the plant species tobacco, rice, and maize (Beetham et al. 1999 Proc.Natl.Acad.Sci.USA 96: 8774-8778; Kochevenko et al. 2003 Plant Phys. 132: 174-184; Okuzaki et al. 2004 Plant Cell Rep. 22: 509-512; Zhu et al. PNAS 1999, 96, 8768). These studies relied upon the introduction of point mutations that confer herbicide resistance. The most tractable system to study TNE has been the yeast *Saccharomyces cerevisiae* (Rice et al. 2001 Mol. Microbiol. 40: 857-868). The use of yeast mutants has identified several genes *(RAD51, RAD52* and *RAD54)* that seem to play a key role in TNE.

Alternative methods of TNE utilize single stranded (ss) oligonucleotides to introduce specific chromosomal mutations. Thus far, ss oligonucleotides have only been tested in yeast and human cells (Liu et al. 2002 Nucl. Acids Res. 30: 2742-2750; review, Parekh-Olmedo et al. 2005 Gene Therapy 12, 639-646*)* .

The efficiency of TNE is increased when human cells are blocked in the S phase (Brachman et al. 2005 DNA Rep. (Amst) 4: 445-457), suggesting that the DNA must have an open configuration for efficient TNE to occur. TNE using ss oligonucleotides has been proposed to occur via a replication mode of gene repair. In this scenario the ss oligonucleotide would anneal at a replication fork and would be assimilated into the daughter strand between the Okasaki fragments of the lagging strand. This results in a mismatch in one of the newly replicated DNA strands. The mutation in the ss oligonucleotide could drive the conversion of the nucleotide in the parental strand, but the likelihood of this directional repair is probably low. This is because current views of mismatch repair during replication promote the notion that the parental strand is used as a template to repair errors in the daughter strand (Stojic et al. 2004 DNA Repair (Amst) 3: 1091-1101). TNE using chimeric oligonucleotides is thought to occur by intercalation of the chimera into the DNA duplex. The chimera RNA strand binds to one strand of the target sequence. This RNA/DNA binding is more stable than a DNA/DNA interaction and may help to stabilize the chimera in the duplex. The gene correction is carried out by the DNA strand of the chimera that binds to the other target strand, resulting in a base change in one strand of the target sequence. After degradation or dissociation of the chimera the resulting mismatch in the target is probably resolved by proteins from the mismatch repair pathway, but this is as yet unconfirmed.

The greatest problem facing the application of TNE in cells of higher organisms such as plants is the low efficiency that has been reported so far. In maize Zhu et al. (2000 Nature Biotech. 18:555-558) reported a conversion frequency of 1 x $10^{-4}$. Subsequent studies in tobacco (Kochevenko et al. 2003 Plant Phys. 132: 174-184) and rice (Okuzaki et al. 2004 Plant Cell Rep. 22: 509-512) have reported frequencies of 1 x $10^{-6}$ and 1 x $10^{-4}$ respectively. These frequencies remain too low for the practical application of TNE.

Faithful replication of DNA is one of the key criteria that mediates maintenance of genome stability and ensures that the genetic information contained in the DNA is passed on free of mutation from one generation to the next. Many errors arise from damage in the parental DNA strand or are generated by agents that react with DNA bases (UV light, environmental toxins). Every organism must maintain a safeguard to prevent or correct these mutations. The mismatch repair system (MMR) is thought to recognize and correct mismatched or unpaired bases caused during DNA replication, in DNA damage surveillance and in prevention of recombination between non-identical sequences (Fedier and Fink, 2004 Int. J Oncol. 2004 ;24(4):1039-47), and contributes to the fidelity of DNA replication in living cells.

MMR recognizes and eliminates misincorporated nucleotides on the newly synthesized strand by an excision/ resynthesis process during replication and thus restores the information contained on the template strand (Jiricny, Mutat Res., 1998,409(3), 107-21; Kolodner and Marsischky, Mol Cell. 1999, 4(3), 439-444; J. Biol. Chem. 1999, 274(38), 26668-26682; Curr. Opin. Genet. Dev., 1999, 9(1), 89-96; Fedier and Fink, 2004 Int J Oncol. 2004 ;24(4):1039-47). This type of replication error occurs spontaneously and is occasionally not detected by 3'-5' exonuclease proofreading activity of the replicative DNA polymerase enzyme complex, or is caused by modified nucleotides in the template strand (Fedier and Fink, 2004 Int J Oncol. 2004 ;24(4):1039-47). Mutations in human MMR proteins have been found to lead to the generation of microsatellite sequences (Fedier and Fink, 2004 Int J Oncol. 2004, 24(4):1039-47). Microsatellites are genetic loci of 1-5 base pair tandem repeats, repeated up to 30 times. These sequences can cause slippage of the DNA polymerase during replication, resulting in the formation of small loop heteroduplexes of one or more nucleotides in the template or nascent DNA strand. Failure to remove these heteroduplexes produces alleles of different sizes in each subsequent round of replication. These alleles have been shown to be present in cancer patients with defective MMR proteins (Peltomaki, J Clin Oncol. 2003, 21(6), 1174-9; Fedier and Fink, 2004 Int J Oncol. 2004, 24(4):1039-47, Jiricny and Nystrom-Lahti, Curr Opin Genet Dev. 2000, 10(2), 157-61).

MMR is also involved in DNA damage signaling via linkage to cell cycle checkpoint activation and apoptosis initiation pathways in the presence of DNA damage (Bellacosa, J Cell Physiol. 2001, 187(2), 137-44.). MMR induces apoptosis to avoid the accumulation of a large number of mutations and thus the absence of the system has been examined in terms of interaction with anti-tumor agents. Patients with compromised MMR systems responded less effectively to treatment with antitumor agents in destruction of tumor cells, thus indicating the importance of the apoptotic induction of the MMR system in drug response (Aquilina and Bignami, J Cell Physiol. 2001 May; 187 (2) :145-54) .

Thus MMR has a dual role in maintaining genomic stability: 1) recognition and correction of mismatches and 2) signaling apoptosis to prevent accumulation of mutations.

The MMR system of bacteria consists of three main proteins, which are referred to as the MutHLS proteins. MutS is an ATPase involved in mismatch recognition. It binds and hydrolyses ATP in the process of binding the mismatched base pairs (Baitinger et al., J Biol Chem. 2003 Dec 5;278(49):49505-11). ATP promotes release of MutS from the mismatch. MutL assists mismatch recognition by initiating and coordinating mismatch repair in the formation of a link between MutS and MutH. The N-terminal domain of this protein is the ATPase domain (Guarne et al., EMBO J. 2004 Oct 27;23(21):4134-45). Each domain of MutL interacts with UvrD helicase to activate UvrD helicase activity and its ability to unwind double stranded DNA to a single strand form. The third protein of the system, MutH binds and nicks DNA with the same recognition sites as MboI and Sau3AI (Baitinger et al., J Biol Chem. 2003 Dec 5;278(49):49505-11; Giron-Monzon et al., Biol Chem. 2004 Nov 19;279(47):49338-45; Joseph et al., DNA Repair (Amst). 2004 Dec 2;3(12): 1561-77). MutH binds any DNA non-specifically in a co-operative and metal-dependent ($Mg2+$) manner (Baitinger et al., J Biol Chem. 2003 Dec 5;278(49):49505-11). The combination and interaction of MutL with ATP produces more specific binding of MutH to fully methylated DNA.

TNE has also been described in a variety of patent applications of Kmiec, *inter alia* in WO0173002, WO03/027265, WO01/87914, WO99/58702, WO97/48714, WO02/10364. In WO 01/73002 it is contemplated that the low efficiency of gene alteration obtained using unmodified DNA oligonucleotides is largely believed to be the result of degradation of the donor oligonucleotides by nucleases present in the reaction mixture or the target cell. To remedy this problem, it is proposed to incorporate modified nucleotides that render the resulting oligonucleotides resistant against nucleases. Typical examples include nucleotides with phosphorothioate linkages, 2'-O-methyl-analogs or locked nucleic acids (LNAs). These modifications are preferably located at the ends of the oligonucleotide, leaving a central DNA domain surrounding the targeted base. Furthermore, the publication stipulates that specific chemical interactions are involved between the converting oligonucleotide and the proteins involved in the conversion. The effect of such chemical interactions to produce nuclease resistant termini using modification other than LNA, phosphorothioate linkages or 2'-O-methyl analogue incorporation in the oligonucleotide is impossible to predict because the proteins involved in the alteration process and their chemical interaction with the oligonucleotide substituents are not yet known and, according to the inventors of WO0173002, cannot be predicted.

In mammals the relationship between DNA methylation is not as simple as in bacteria. Following DNA replication, mammalian DNA possesses a transient, strand-specific CpG hemi-methylation in the parental strand (Drummond and Bellacosa, Nucleic Acids Res. 2001 Jun 1; 29(11):2234-43.). Maintenance cytosine methyltransferases then restore full methylation to hemi-methylated CpG sites.

It is also known that in systems where both strands of the acceptor DNA are de-methylated, no preference exist for the donor strand or the mismatch-carrying acceptor strand. Either nucleotide is replaced with equal probability. (see for instance Alberts et al. in "Molecular Biology of the Cell", 2nd edition, 1989, Garland publishing, pp234ff)

As the efficiency of the current methods of ODTNE is relatively low (as stated previously, between $10^{-6}$ and $10^{-4}$, despite reported high delivery rates of the oligonucleotide of 90%) there is a need in the art to come to methods for TNE that are more efficient. Accordingly, the present inventors have set out to improve on the existing TNE technology.

### *Description of the invention*

The present inventors have now found that by incorporating nucleotides into the donor oligonucleotide for TNE that are capable of binding more strongly to the acceptor DNA than the corresponding unmodified nucleotides like A,C, T, or G, the rate of TNE can be increased significantly. Without being bound by theory, the present inventors believe that by the incorporation of modified nucleotides into the donor oligonucleotide, the donor oligonucleotide binds more strongly to the acceptor DNA and hence increases the ratio of TNE. The present inventors have also now found that the use of demethylated genomic DNA in the cells to be used for TNE, optionally in combination with the use of synthetic oligonucleotides that (partly) comprise modified nucleotides with improved binding capacity provides for an improved method for performing TNE.

Demethylation of genomic DNA can be achieved through chemical treatments of the biological material prior to DNA replication or through the use of methylation-deficient mutants. Both methods result in demethylated parental DNA strands. The use of demethylated genomic DNA alone, or the use of modified synthetic oligonucleotides alone, or the combination of both, will result in a duplex or triplex DNA structure wherein the removal of the mismatched nucleotide in the parental strand, and the subsequent stable incorporation in the genome of the deliberate mismatched base designed in the oligonucleotide is achieved with high efficiency.

The present invention is also based on the inventive consideration that the desired targeted nucleotide exchange can be achieved by the use of (partly) modified oligonucleotides. The location, type and amount of modification (i.e. status) of the oligonucleotide can be varied as will be disclosed herein below.

The present invention thus, in one aspect provides ((fully) modified) oligonucleotides. The oligonucleotides can be used to introduce specific genetic changes in plant and animal or human cells. The invention is applicable in the field of biomedical research, agriculture and to construct specifically mutated plants and animals, including humans. The invention is also applicable in the field of medicine and gene therapy.

The sequence of an oligonucleotide of the invention is homologous to the target strand except for the part that contains a mismatch base that introduces the base change in the target strand. The mismatched base is introduced into the target sequence. By manipulating the modification (compared to conventional A, C, T, or G) of the nucleotides, and more in particular, by manipulating the type, location and amount of modification of the oligonucleotide that introduces the mismatch and/or by manipulating the degree of methylation of one or both strands of the DNA duplex in which the oligonucleotide can intercalate, the efficiency (or the degree of successful incorporation of the desired nucleotide at the desired position in the DNA duplex) can be improved.

Another aspect of the invention resides in a method for the targeted alteration of a parent DNA strand (first strand, second strand) by contacting the parent DNA duplex with an oligonucleotide that contains at least one mismatch nucleotide compared to the parent strand, wherein the donor oligonucleotide contains a section that is modified to have a higher binding capacity than the parent (acceptor)strand and/or wherein the parent strand is methylated to a lower degree of methylation, in the presence of proteins that are capable of targeted nucleotide exchange.

Thus, the inventive gist of the invention lies in the improvement in the binding capacity, of the intercalating oligonucleotide (sometimes referred to as the donor) relative to the unmodified intercalating oligonucleotide and/or the modification (de-methylation) of one or both the strand(s) of the DNA duplex (sometimes referred to as the acceptor strand).

***Detailed description of the invention***

In one aspect, the invention pertains to an oligonucleotide for targeted alteration of a duplex DNA sequence. The duplex DNA sequence contains a first DNA sequence and a second DNA sequence. The second DNA sequence is the complement of the first DNA sequence and pairs to it to form a duplex. The oligonucleotide comprises a domain that comprises at least one mismatch with respect to the duplex DNA sequence to be altered. Preferably, the domain is the part of the oligonucleotide that is complementary to the first strand, including the at least one mismatch.

Preferably, the mismatch in the domain is with respect to the first DNA sequence. The oligonucleotide comprises a section that is modified (contains alternative or modified nucleotides) to have a higher binding affinity than the (corresponding part of the) second DNA sequence. In a certain embodiment, the second DNA sequence is methylated to a lower degree than the (corresponding part of the) section on the oligonucleotide. In certain embodiments, the acceptor strand and/or the oligonucleotide are not methylated or are methylated to the same extent, such that no distinction can be made based on methylation between the donor strand and the parental strand. This may remove any strand bias and render the targeted nucleotide exchange to a statistical process as any mechanism involving methylation can no longer distinguish between the two strands. In this embodiment any TNE will evolve from the effect of the modified nucleotides (wherein modification is not equal to methylation) that are incorporated in the oligonucleotide.

The domain that contains the mismatch and the section containing the modified nucleotide(s) may be overlapping. Thus, in certain embodiments, the domain containing the mismatch is located at a different position on the oligonucleotide than the section of which the modification is considered. In certain embodiments, the domain incorporates the section. In certain embodiments the section can incorporate the domain. In certain embodiments the domain and the section are located at the same position on the oligonucleotide and have the same length i.e. they coincide in length and position. In certain embodiments, there can be more than one section within a domain.

For the present invention, this means that the part of the oligonucleotide that contains the mismatch which is to be incorporated in the DNA duplex can be located at a different position from the part of the oligonucleotide. In particular, in certain embodiments wherein the cell's repair system (or at least the proteins involved with this system, or at least proteins that are involved in TNE) determines which of the strands contain the mismatch and which strand is to be used as the template for the correction of the mismatch.

In certain embodiments, the oligonucleotide comprises a section that contains at least one, preferably at least 2, more preferably at least 3 modified nucleotide(s) and/or wherein the second strand contains at least one, preferably at least 2, more preferably at least 3 methylated nucleotide(s) less than the corresponding part of the section. In certain embodiments, the section on the oligonucleotide can contain more than 4, 5, 6, 7, 8, 9, or 10 modified nucleotides. In certain embodiments the section is fully modified. In certain embodiments the second strand is not methylated, at least at the position (or over the length of the section complementary to the first strand) of the oligonucleotide.

In certain embodiments, more than one mismatch can be introduced, either simultaneously or successively. The oligonucleotide can accommodate more than one mismatch on either adjacent or removed locations on the oligonucleotide. In certain embodiments the oligonucleotide can comprise two, three, four or more mismatch nucleotides which may be adjacent or remote (i.e. non-adjacent). The oligonucleotide can comprise further domains and sections to accommodate this, and in particular can comprise several sections. In certain embodiments, the oligonucleotide may incorporate a potential insert that is to be inserted in the acceptor strand. Such an insert may vary in length from more than five up to 100 nucleotides. In a similar way in certain embodiments, deletions can be introduced of similar length variations (from 1 to 100 nucleotides).

In a further aspect of the invention, the design of the oligonucleotide can be achieved by:

determining the sequence of the acceptor strand, or at least of a section of the sequence around the nucleotide to be exchanged. This can typically be in the order of at least 10, preferably 15, 20, 25 or 30 nucleotides adjacent to the mismatch, preferably on each side of the mismatch, (for example GGGGGGXGGGGGG, wherein X is the mismatch);

designing a donor oligonucleotide that is complementary to one or both the sections adjacent to the mismatch and contains the desired nucleotide to be exchanged (for example CCCCCCYCCCCCC);

providing (e.g. by synthesis) the donor oligonucleotide with modifications at desired positions. Modifications may vary widely, depending on the circumstances. Examples are $C^mC^mC^mC^mC^mC^mYC^mC^mC^mC^mC^mC^m$, $C^mCC^mCC^mCYC^mCC^mCC^mC$, $CCCCCCYC^mC^mC^mC^mC^mC^m$, $C^mC^mC^mC^mC^mC^mYCCCCCC$, $CCCCCC^mYC^mCCCCC$, $C^mCCCCCYC^mCCCCC$, $C^mCCCCCYCCCCCC^m$, $C^mCCCCCYCCCCCC$, and so on, wherein $C^m$ stands for a modified nucleotide residue. For a different acceptor sequence, e.g. ATGCGTACXGTCCATGAT, corresponding donor oligonucleotides can be designed, e.g. TACGCATGYCAGGTACTA with modification as variable as outlined hereinbefore.

subjecting the DNA to be modified with the donor oligonucleotide in the presence of proteins that are capable of targeted nucleotide exchange, for instance, and in particular, proteins that are functional in the mismatch repair mechanism of the cell.

The delivery of the oligonucleotide can be achieved via electroporation or other conventional techniques that are capable of delivering either to the nucleus or the cytoplasm. In vitro testing of the method of the present invention can be achieved using the Cell Free system as is described i.a. in WO01/87914, WO03/027265, WO99/58702, WO01/92512.

As used herein, the capability of the donor oligonucleotide to influence the TNE depends on the type, location and amount of modified nucleotides that are incorporated in the donor oligonucleotide. This capability can be quantified for instance by normalising the binding affinity (or the binding energy (Gibbs Free Energy)) between conventional nucleotides at 1, i.e. for both AT and GC bindings, the binding affinity is normalised at 1. For the oligonucleotides of the present invention the Relative Binding Affinity (RBA) of each modified nucleotide is > 1. This is exemplified in a formula below:

$$RBA = \sum_{n}^{1} RBA(modified) - \sum_{m}^{1} RBA(unmodified) > 0$$

Wherein RBA is the total relative binding affinity, RBA(modified) is the sum of the relative binding affinity of the modified oligonucleotide with a length of n nucleotides and RBA(unmodified) is the sum of the relative binding affinity of the unmodified oligonucleotide with a length of m nucleotides. For example, an 100 bp oligonucleotide contains 10 modifications, each with a relative binding affinity of 1.1. The total RBA then equals: RBA = [(10*1.1) + (90*1.0)] - (100*1.0) = 1.

Note that the definition of RBA is in principle independent of the length of the nucleotide strand that is compared. However, when RBAs of different strands are compared it is preferred that the strands have about the same length or that sections of comparable length are taken. Note that RBA does not take into account that modification can be grouped together on a strand. A higher degree of modification of a certain strand A compared to a strand B thus means that RBA (A) > RBA(B). For upstream and downstream sections, corresponding RBA values may be defined and used. To accommodate the effect of the position of the modified nucleotide a weighing factor can be introduced into the RBA value. For instance, the effect of a modified nucleotide on the donor oligonucleotide adjacent to the mismatch can be larger than that of a modified nucleotide that is located at a distance five nucleotides removed from the mismatch. In the context of the present invention, RBA (Donor)> RBA (Acceptor).

In certain embodiments, the RBA value of the Donor may be at least 0.1 larger than the RBA of the Acceptor. In certain embodiments, the RBA value of the Donor may be at least 0.2, 0.3, 0.4, 0.5, 0.6, 0.7, 0.8, 0.9, 1.0, 1.5, 2.0, 2.5 larger than the RBA of the Acceptor. RBA values can be derived from conventional analysis of the modified binding affinity of the nucleotide, such as by molecular modelling, thermodynamic measurements etc. Alternatively they can be determined by measurement of Tm differences between modified and unmodified strands. Alternatively, the RBA can be expressed as the difference in Tm between the unmodified and the modified strand, either by measurement or by calculation using conventional formulates for calculating the Tm of a set of nucleotides, or by a combination of calculation and measurements.

The donor oligonucleotides according to the invention contains modifications to improve the hybridisation characteristics such that the donor exhibits increased affinity for the target DNA strand so that intercalation of the donor is easier. The donor oligonucleotide can also be modified to become more resistant against nucleases, to stabilize the triplex or quadruplex structure. Modification of the donor oligonucleotides can comprise phosphorothioate modification, 2-OMe substitutions, the use of LNAs (Locked nucleic acids), PNAs (Peptide nucleic acids), ribonucleotide and other bases that modifies, preferably enhances, the stability of the hybrid between the oligonucleotide and the acceptor stand.

Particularly useful among such modifications are PNAs, which are oligonucleotide analogues where the deoxyribose backbone of the oligonucleotide is replaced by a peptide backbone. One such peptide backbone is constructed of repeating units of N- (2-aminoethyl) glycine linked through amide bonds. Each subunit of the peptide backbone is attached to a nucleobase (also designated "base"), which may be a naturally occurring, non-naturally occurring or modified base. PNA oligomers bind sequence specifically to complementary DNA or RNA with higher affinity than either DNA or RNA. Accordingly, the resulting PNA/DNA or PNA/RNA duplexes have higher melting temperatures (Tm). In addition, the Tm of the PNA/DNA or PNA/RNA duplexes is much less sensitive to salt concentration than DNA/DNA or DNA/RNA duplexes. The polyamide backbone of PNAs is also more resistant to enzymatic degradation. The synthesis of PNAs is described, for example, in WO 92/20702 and WO 92/20703, the contents of which are incorporated herein by reference in their entireties. Other PNAs are illustrated, for example, in WO93/12129 and United States Patent No. 5,539,082, issued July 23,1996, the contents of which are incorporated herein by reference in their entireties. In addition, many scientific publications describe the synthesis of PNAs as well as their properties and uses. See, for example, Patel, Nature, 1993, 365, 490 ; Nielsen et al., Science, 1991, 254, 1497; Egholm, J. Am. Chem. Soc., 1992,114,1895; Knudson et al., Nucleic Acids Research, 1996,24,494; Nielsen et al., J. Am. Chem. Soc., 1996,118,2287 ; Egholm et al., Science, 1991,254,1497;Egholm et al., J. Am. Chem. Soc., 1992,114,1895; and Egholm et al., J Am. Chem. Soc., 1992,114,9677.

Useful modifications are also known as Super A and Super T , obtainable from Epoch Biosciences Germany. These modified nucleotides contain an additional substituent that sticks into the major groove of the DNA where it is believed to improve base stacking in the DNA duplex. See also Fig 4

Useful modifications also include one or more monomers from the class of synthetic molecules known as locked nucleic acids (LNAs). LNAs are bicyclic and tricyclic nucleoside and nucleotide analogues and the oligonucleotides that contain such analogues. The basic structural and functional characteristics of LNAs and related analogues are disclosed in various publications and patents, including WO 99/14226, WO 00/56748, WO00/66604, WO 98/39352, United States Patent No.6, 043, 060, and United States Patent No. 6,268,490, all of which are incorporated herein by reference in their entireties.

The donor oligonucleotides can also be made chimeric, i.e. contain sections of DNA, RNA, LNA, PNA or combinations thereof.

Thus, in certain embodiments, the oligonucleotide further contains other, optionally non-methylated, modified nucleotides.

In certain embodiments, the oligonucleotide is resistant against nucleases. This is advantageous to prevent the oligonucleotide from being degraded by nucleases and enlarges de chance that the donor oligonucleotide can find its target (acceptor molecule).

The table I below present a selection of nucleotides that express an increased DNA binding affinity and /or increased nuclease degradation resistance.

| **Modified Nucleotide** | **Effect on DNA binding** | **Effect on nuclease resistance** |
|---|---|---|
| **nuclease resistant** | | |
| Morpholino phosphoroamidate | | + |
| amino modifications (3' or 5') | | + |
| Phosphorothioate bond (internal, | | + |
| 2'-fluoroarabinonucleic acid | | + |
| **nuclease resistant and high binding affinity** | | |
| Methoxiacridine | ++ | + |
| Cyclohexene nucleic acids (CeNAs) | +/- | + |
| Methylphosphonates | + | ++ |
| α-L-LNA | + | ++ |
| 2'-O-methyl-2-aminopurine | + | + |
| 2'-O-methyl-2,6-diaminopurine | + | + |
| 2'-O-methyl-3-deaza-5-azacytidine | + | + |
| 2'-O-methyl-5-fluorouridine | + | + |
| 2'-O-methyl-5-methylcytidine | + | + |
| 2'-O-methyl-5-methyluridine | + | + |
| 2'-O-methylinosine | + | + |
| 2'-fluoro-RNA (C and U) | + | + |

(continued)

| | | |
|---|---|---|
| **nuclease resistant and high binding affinity** | | |
| β-D-LNA | + | + |
| 2'-O-methoxyethyl RNA (MOE) | + | + |
| 2'-O-alkyl RNA | + | + |
| $N^3$-$P^S$-phosphoramidate (NP) | + | + |
| tricyclo-DNA | + | + |
| **binding affinity** | | |
| 2-amino-adenosine | + | |
| 2,6-diaminopurine-2'-deoxyriboside | + | |
| 5-propynyl-2'-deoxynucleoside (C | + | |
| 5-methyl-isodeoxycytidine | + | |
| 5-methyl-2'-deoxycytidine | + | |
| $N^4$-ethyl-2'-deoxycytidine | + | |
| superA and superT | + | |

Phosphorothioate bonds

Many of the nucleotide modifications commercially available have been developed for use in antisense applications for gene therapy. The simplest and most widely used nuclease-resistant chemistry available for antisense applications (the "first generation" antisense-oligonucleotide) is the phosphorothioate (PS) modification. In these molecules, a sulfur atom replaces a non-bridging oxygen in the oligonucleotide phosphate backbone (Fig. 3a), resulting in resistance to endonuclease and exonuclease activity. For gene therapy, a phosphorothioate/phosphodiester chimera generally has one to four PS-modified internucleoside linkages on both the 5'- and 3'-ends with a central core of unmodified DNA. The phosphorothioate bonds can be incorporated, however, at any desired location in the oligonucleotide.

6-Chloro-2-methoxiacridine

Oligonucleotides bearing the 6-chloro-2-methoxiacridine molecule at either terminus or within the sequence have the ability to intercalate efficiently into a double helix. The 6-chloro-2-methoxiacridine group is coupled to one of the free hydroxylgroups of the phosphate backbone (Fig. 3c). Acridine-labeled oligonucleotides can thus be used in applications where increased stability of oligonucleotides hybrids is crucial. Adding the modification to the 3' terminus also protects the oligonucleotide from exonuclease degradation. These oligonucleotides possess the ability to form triplex helices, and, due to their relative hydrophobicity, to pass membranes more easily than normal oligonucelotides.

Methylphosphonates

Methylphosphonates are nucleic acid analogs which contain methylphosphonate linkages instead of the naturally occurring negatively charged phosphodiester bonds (Fig. 3b). These analogs are non-ionic and hydrophobic and are taken up by mammalian cells via passive diffusion. Their melting temperatures are less sensitive to salt conditions. They have been found to be effective antisense reagents, capable of specifically controlling viral or cellular gene expression at the mRNA level. Methylphosphonates form duplex hybrids with complementary nucleic acids by standard Watson-Crick base pairing interactions. The methylphosphonate backbone is completely resistant to nuclease degradation.

Locked nucleic acids

Locked Nucleic Acid (LNA) is a DNA analogue with very interesting properties for use in antisense gene therapy. Specifically, it combines the ability to discriminate between correct and incorrect targets (high specificity) with very high bio-stability (low turnover) and unprecedented affinity (very high binding strength to target). In fact, the affinity increase recorded with LNA leaves the affinities of all previously reported analogues in the low-to-modest range.

LNA is an RNA analogue, in which the ribose is structurally constrained by a methylene bridge between the 2'-oxygen and the 4'-carbon atoms (Fig. 4). This bridge restricts the flexibility of the ribofuranose ring and locks the structure into a rigid bicyclic formation. This so-called N-type (or 3'-endo) conformation results in an increase in the $T_m$ of LNA containing duplexes, and consequently higher binding affinities and higher specificities. NMR spectral studies have actually demonstrated the locked N-type conformation of the LNA sugar, but also revealed that LNA monomers are able to twist their unmodified neighbour nucleotides towards an N-type conformation. Importantly, the favourable characteristics of LNA do not come at the expense of other important properties as is often observed with nucleic acid analogues.

LNA can be mixed freely with all other chemistries that make up the DNA analogue universe. LNA bases can be incorporated into oligonucleotides as short all-LNA sequences or as longer LNA/DNA chimeras. LNAs can be placed in internal, 3' or 5'-positions. However, due to their rigid bicyclic conformations, LNA residues sometimes disturb the helical twist of nucleic acid strands. It is hence generally less preferred to design an oligonucleotide with two or more adjacent LNA residues. Preferably, the LNA residues are separated by at least one (modified) nucleotide that does not disturb the helical twist, such as a conventional nucleotide (A, C, T, or G).

The originally developed LNA monomer (the ß-D-oxy-LNA monomer) has been modified into new LNA monomers (Fig. 4). The novel α-L-oxy-LNA shows superior stability against 3' exonuclease activity, and is also more powerful and more versatile than ß-D-oxy-LNA in designing potent antisense oligonucleotides. Also xylo -LNAs and L-ribo LNAs can be used, as disclosed in WO9914226, WO00/56748, WO00/66604

Methylated, propynylated and aminated nucleotides

Methylation of the cytosine $C^5$ atom is the naturally occurring DNA C-methylation (5'-$^m$CG-3' and 5'-$^m$CNG-3' in plants, and 5'-C$^m$CNGG-3' in prokaryotes). Apart from the biological functions of methylation, its chemical effect is a rise in the $T_m$ of the duplex formation by 1.3°C per methylated cytosine. Such methylated C-residues (5-methyl-deoxycytosine, Fig. 5a) could be incorporated in oligonucleotides solely to increase their duplex stability.

Even better thermal stability is obtained with a propynyl group (3-C-chain with a triple bond) at the $C^5$ position (Fig. 5b). A single $C^5$-propynyl-cytosine residue increases the $T_m$ by 2.8°C, a single $C^5$-propynyl-thymidine by 1.7°C.

Another commonly described nucleotide modification (the "second generation" of antisense oligonucleotide) is the 2'-O methylation of RNA residues. This methylation provides resistance to exonuclease activity and can be applied to any of the RNA nucleotides. In Fig. 5c, a double-methylated molecule is depicted, carrying methyl groups at both the ribosyl 2'-O and the cytosine $C^5$. This molecule, 2'-O-methyl-5-methyl-ribocytidine (2'-OMe-5-Me-C) has both increased binding affinity and exonuclease resistance

The highest thermal stability increase has been described for 2'-aminated adenosine residues. These so-called 2-amino-A molecules (Fig. 5d) carry an extra $NH_2$ group at the outward facing ring, allowing adenine to form three, not two, hydrogen bonds with thymidine, which increases the $T_m$ by 3.0°C for each single 2-amino-A residue.

Cyclohexene nucleic acids

Cyclohexene nucleic acids (CeNAs) are nucleotides in which the five-membered furanose ring has been replaced by a six-membered ring (Fig. 6). This results in a high degree of conformational rigidity of the oligomers. CeNAs form stable duplexes with RNA and DNA and protect against nucleolytic degradation. Because the binding affinity of CeNAs to RNA is much better than to DNA, and because CeNA/RNA duplexes activate RNase H degradation, this molecule is a favourite for therapeutic antisense studies.

In certain embodiments of the invention, the nucleotide in the oligonucleotide at the position of the mismatch can be modified. Whether or not the mismatch can be modified will depend to a large extent on the exact mechanism of the targeted nucleotide exchange or of the cell's DNA repair mechanism using the difference in affinity between the donor and acceptor strands. The same holds for the exact location of the other modified positions in the neighbourhood or vicinity of the mismatch. However, based on the disclosure presented herein, such an oligonucleotide can be readily designed and tested, taking into account the test procedures for suitable oligonucleotides as described herein elsewhere. In certain embodiments, the nucleotide at the position of the mismatch is not modified. In certain embodiments, modification is adjacent to the mismatch, preferably within 2, 3, 4, 5, 6 or 7 nucleotides of the mismatch. In certain embodiments, modification is located at a position downstream from the mismatch. In certain embodiments, modification is located at a position upstream from the mismatch. In certain embodiments, the modification is located from 10 bp to 10kB from the mismatch, preferably from 50 to 5000 bp, more preferably from 100 to 500 from the mismatch.

The oligonucleotides that are used as donors can vary in length but generally vary in length between 10 and 500 nucleotides, with a preference for 11 to 100 nucleotides, preferably from 15 to 90, more preferably from 20 to 70 most preferably from 30 to 60 nucleotides.

In one aspect, the invention pertains to a method for the targeted alteration of a duplex acceptor DNA sequence, comprising combining the duplex acceptor DNA sequence with a donor oligonucleotide, wherein the duplex acceptor DNA sequence contains a first DNA sequence and a second DNA sequence which is the complement of the first DNA sequence and wherein the donor oligonucleotide comprises a domain that comprises at least one mismatch with respect to the duplex acceptor DNA sequence to be altered, preferably with respect to the first DNA sequence, and wherein a section of the donor oligonucleotide is modified to express a higher degree of affinity to the first DNA sequence compared to an unmodified nucleotide at that position in the oligonucleotide and/or wherein the second DNA is methylated to a lower degree of methylation than the corresponding section of the donor oligonucleotide, in the presence of proteins that are capable of targeted nucleotide exchange.

The invention is, in its broadest form, generically applicable to all sorts of organisms such as humans, animals, plants, fish, reptiles, insects, fungi, bacteria and so on. The invention is applicable for the modification of any type of DNA, such as DNA derived from genomic DNA, linear DNA, artificial chromosomes, nuclear chromosomal DNA, organelle chromosomal DNA, BACs, YACs. The invention can be performed *in vivo* as well as *ex vivo.*

The invention is, in its broadest form, applicable for many purposes for altering a cell, correcting a mutation by restoration to wild type, inducing a mutation, inactivating an enzyme by disruption of coding region, modifying bioactivity of an enzyme by altering coding region, modifying a protein by disrupting the coding region.

The invention also relates to the use of oligonucleotides essentially as described hereinbefore, for altering a cell, correcting a mutation by restoration to wild type, inducing a mutation, inactivating an enzyme by disruption of coding region, modifying bioactivity of an enzyme by altering coding region, modifying a protein by disrupting the coding region, mismatch repair, targeted alteration of (plant)genetic material, including gene mutation, targeted gene repair and gene knockout

The invention further relates to kits, comprising one or more oligonucleotides as defined herein elsewhere, optionally in combination with proteins that are capable of inducing MRM, and in particular that are capable of TNE.

The invention further relates to modified genetic material obtained by the method of the present invention, to cells and organisms that comprise the modified genetic material, to plants or plant parts that are so obtained.

In certain embodiments, the genomic DNA can be demethylated prior to TNE, for instance by subjecting the genomic DNA, or cultures comprising genomic DNA or other biological material that contains the genomic DNA that is to be subjected to TNE, to conventional demethylation treatments, such as methods using azacytidine.

In certain embodiments of the present invention, it has been found that significant increases (200-800%) in the rate of targeted nucleotide exchange have been achieved when the oligonucleotide comprises one or more LNA residues, preferably one LNA residue, located at a distance of at least one base pair from the mismatch. Preferably the oligonucleotide comprises two or more, preferably two LNAs, located at either side of the mismatch, preferably each independently located at a distance of at least one base pair from the mismatch. In other words, in preferred embodiments of the invention, at least one, preferably one, LNA is located at each side of the mismatch, preferably and independently at a position of at least one base pair from the mismatch. Schematically this can be depicted as: L-N-M-N-L, wherein L= one or more LNA residues, preferably one; N is, independently on each side of M, one normal nucleotide (A, C, T or G) or a dinucleotide (i.e. two nucleotides) or a trinucleotide (i.e. three nucleotides); M representing the position of the mismatch. Thus, in certain preferred embodiments, the LNA residues flanking the mismatch are located 3 or 4 basepairs from each other.

In certain other embodiments of the invention, it has been found that significant increases in the rate of targeted nucleotide exchange have been achieved using one or more 5-propynylated nucleotides. The propynylated nucleotide may be located adjacent to the mismatch or may be located at a distance of at least one base pair from the mismatch. Preferably the oligonucleotide comprises two or more, preferably two 5-propynylated nucleotides, that are preferably located at either side of the mismatch, preferably each independently located adjacent or at a distance of at least one base pair from the mismatch. In other words, in preferred embodiments of the invention, at least one, preferably one, 5-propynylated nucleotide is located at each side of the mismatch, preferably and independently at a position adjacent to the mismatch or ate a distance of at least one base pair from the mismatch. Schematically this can be depicted as: P-N-M-N-P, wherein P= one or more 5-propynylated nucleotide residues, preferably one 5-propynylated nucleotide; N is, independently on each side of M, zero or one normal nucleotide (A, C, T or G) or a dinucleotide (i.e. two nucleotides) or a trinucleotide (i.e. three nucleotides); M representing the position of the mismatch. Thus, in certain preferred embodiments, the 5-propynylated nucleotides residues flanking the mismatch are located one, (i.e. the mismatch itself), 2, 3 or 4 basepairs from each other. Certain preferred 5-propynylated nucleotides are 5-propynyl-2-deoxycytidine and/or 5-propynyl-2-deoxyuracil. The 5-propynylated nucleotides may be used in combination, i.e. in certain embodiments, the oligonucleotide may contain either 5-propynyl-2-deoxycytidine alone, 5-propynyl-2-deoxyuracil alone or a combination of both.

In certain other embodiments of the invention, it has been found that significant increases in the rate of targeted nucleotide exchange have been achieved using N4-ethyl-2'-deoxycytidine.

The N4-ethyl-2'-deoxycytidine may be located adjacent to the mismatch or may be located at a distance of at least one base pair from the mismatch. Preferably the oligonucleotide comprises two or more, preferably two N4-ethyl-2'-deoxycytidine, that are preferably located at either side of the mismatch, preferably each independently located adjacent or at a distance of at least one base pair from the mismatch. In other words, in preferred embodiments of the invention, at least one, preferably one, N4-ethyl-2'-deoxycytidine is located at each side of the mismatch, preferably and independently at a position adjacent to the mismatch or at a distance of at least one base pair from the mismatch.

Schematically this can be depicted as: D-N-M-N-D, wherein D= one or more N4-ethyl-2'-deoxycytidine residues, preferably one N4-ethyl-2'-deoxycytidine residue; N is, independently on each side of M, zero or one normal nucleotide (A, C, T or G) or a dinucleotide (i.e. two nucleotides) or a trinucleotide (i.e. three nucleotides); M representing the position of the mismatch. Thus, in certain preferred embodiments, the N4-ethyl-2'-deoxycytidine residues flanking the mismatch are located one, (i.e. the mismatch itself), 2, 3 or 4 basepairs from each other.

Without being bound by theory, it is applicant's belief that the efficiency of TNE can be improved by incorporating novel nucleotides in the oligonucleotide that have altered properties compared with normal DNA, as stated herein elsewhere. The properties considered important for TNE are: (1) nuclease resistance, so that the oligonucleotide remains intact in the plant cell, and (2) high binding affinity, thus increasing the likelihood that an oligonucleotide finds and remains bound to its homologous target. One other parameter that is also considered as being of importance is minimizing the oligonucleotide secondary structure. Small complementary regions within the oligonucleotide itself can lead to hairpin-loop structures that may prevent oligonucleotide transport to the nucleus, the oligo finding its target or the TNE reaction itself.

<u>Description of the Figures</u>

**Figure 1:** Schematic representation of targeted nucleotide exchange. An acceptor duplex DNA strand containing a nucleotide that is to be exchanged (X) is brought into contact with a donor oligonucleotide (schematically given as $NNN^m$-$NNN^m$-$YNN^m$-$NN^m$-) containing the nucleotide to be inserted (Y). The triplex structure is subjected to or brought into contact with an environment that is capable of TNE or at least with proteins that are capable of performing TNE, such as are known as the cell-free enzyme mixture or a cell-free extract (see i.a. WO99/58702, WO01/73002).

**Figure 2:** Chemical structures of some deoxyribocytidine analogues: (a) phosphorothioate bond; (b) methylphosphonate; (c) 5' terminal nucleotide residue coupled to a 6-chloro-2-methoxiacridine group.

**Figure 3:** Chemical structures of different diastereoisomers of Locked Nucleic Acids presented as cytosine nucleotides: ß-D-oxy-LNA, the original LNA structure with a 2'-O-4'-C methylene bridge in "ß" configuration of the $C^4$ atom; (b) ß-D-thio-LNA, with an S-atom in the methylene bridge; α-L-oxy-LNA, containing the 2'-O-4'-C methylene bridge in the "α" position of $C^4$

**Figure 4:** Structure of Super A and Super T

**Figure 5:** Chemical structures of (a) 5-methyl-deoxycytosine; (b) 5-propynyl-deoxycytosine; (c) 2'-O-methyl-5-methyl-cytosine; (d) 2-amino-adenosine.

**Figure 6:** Chemical structure of cyclohexene nucleic acid (CeNA).

clauses

Aspects of the invention can be summarized by the following Lauses

1. An oligonucleotide for targeted alteration of a duplex DNA sequence, the duplex DNA sequence containing a first DNA sequence and a second DNA sequence which is the complement of the first DNA sequence, the oligonucleotide comprising a domain that is capable of hybridizing to the first DNA sequence, which domain comprises at least one mismatch with respect to the first DNA sequence, and wherein the oligonucleotide comprises at least one section that contains at least one modified nucleotide having a higher binding affinity compared to naturally occurring A, C, T or G and wherein the at least one modified nucleotide binds stronger to a nucleotide in an opposite position in the first DNA sequence as compared to a naturally occurring nucleotide complementary to the nucleotide in the opposite position in the first DNA sequence.

2. Oligonucleotide according to clause 1, wherein the oligonucleotide comprises at least 2 sections, preferably at least 3 sections that independently contain at least one, preferably at least 2, more preferably at least 3, 4, 5, 6, 7, 8, 9 or 10, modified nucleotides.

3. Oligonucleotide according to clause 1 or 2, wherein the sections are located near or at the 3'-end, the 5'-end and/or encompass the position of the mismatch.

4. Oligonucleotide according to clauses 1-3, wherein the nucleotide at the position of the mismatch is not modified.

5. Oligonucleotide according to clauses 1-4, wherein the at least one modified nucleotide is located adjacent to the mismatch, preferably within 2, 3, 4, 6, 7, 8, 9, or 10 nucleotides of the mismatch.

6. Oligonucleotide according to clauses 1-5, having a length from 10 to 500 nucleotides.

7. Oligonucleotide according to clauses 1-6, wherein the (modified) section is the domain.

8. Oligonucleotide according to clauses 1-7, wherein the at least one modified nucleotide is selected from the group consisting of backbone modified nucleotides and/or base modified nucleotides.

9. Oligonucleotide according to clauses 1-8, wherein the nucleotide is selected from the group consisting of

a. cyclohexene nucleic acids (CeNAs);
b. locked nucleic acids (LNAs);
c. peptide nucleic acids (PNAs);
d. 2'-O-methyl substituted nucleotides;
e. methylphosphonate substituted nucleotides;
f. 6-chloro-2-methoxiacridine substituted nucleotides;
g. 2'-fluoro-RNAs;
h. 2'-O-methoxyethyl-RNAs;
i. 2'-O-alkyl-RNAs;
j. tricyclo-DNA;
k. N3-P5-phosphoramidate substituted nucleotides;
l. 2, 6-diaminopurine based nucleotides;
m. Methylated, propynylated and aminated nucleotides;
n. Super A en Super T;

10. Oligonucleotide according to clauses 1-9, wherein the modified nucleotide is selected from the group consisting of:

alpha-L-LNA;
Beta-D-Oxy-LNA;
Beta-D-thio-LNA;
alpha-L-oxy-LNA;
2'-O-methyl-2-aminopurine;
2'-O-methyl-2, 6-diaminopurine;
2'-O-methyl-3-deaza-5-azacytidine;
2'-O-methyl-5-fluorouridine;
2'-O-methyl-5-methylcytidine;
2'-O-methyl-5-methyluridine;
2'-O-methylinosine;
2'-fluoro-ribocytidine;
2'-fluoro-ribouridine;
2'-O-methoxyethyl-ribocytidine;
2'-O-methoxyethyl-ribouridine;
2'-O-methoxyethyl-riboadenosine;
2'-O-methoxyethyl-riboguanine;
2'-O-alkyl-ribocytidine;
2'-O-alkyl-ribouridine;
2'-O-alkyl-riboadenosine;
2'-O-alkyl-riboguanine;
2-amino-adenosine;
2, 6-diaminopurine-2'-deoxyriboside;
5-propynyl-2'-deoxycytidine
5-propynyl-2'-deoxyuridine
5-methyl-isodeoxycytidine;
5-methyl-2'-deoxycytidine;
$N^4$-ethyl-2'-deoxycytidine.

11. Oligonucleotide according to clauses 1-10, wherein the oligonucleotide comprises nuclease resistant nucleotides.

12. Oligonucleotide according to clause 11, wherein the nuclease resistant nucleotides are phosphorothioate modified nucleotides such that the oligonucleotide comprises at least one, two or preferably at least three phosphorothioate linkages in the oligonucleotide.

13. Oligonucleotide according to clause 11, wherein the nuclease resistant nucleotides are 2'-O methyl-substituted nucleotides.

14. Oligonucleotide according to clause 11, wherein the nuclease resistant nucleotides are LNAs.

15. Oligonucleotide according to clauses 1-10, wherein the oligonucleotide comprises one or more LNA residues located at a distance of at least one base pair from the mismatch.

16. Oligonucleotide according to clause 15, wherein one LNA is located at each side of the mismatch at a position of at least one base pair from the mismatch.

17. Oligonucleotide according to clauses 1-10, wherein the oligonucleotide comprises one or more 5-propynylated nucleotides and or N4-ethyl-2'-deoxycytidine located adjacent to or at a distance of at least one base pair from the mismatch.

18. Oligonucleotide according to clause 15, wherein one 5-propynylated nucleotide or N4-ethyl-2'-deoxycytidine is located at each side of the mismatch adjacent to or at a position of at least one base pair from the mismatch.

19. Oligonucleotide according to clauses 17-18, wherein the 5-propynylated nucleotide are 5-propynyl-2-deoxycytidine and/or 5-propynyl-2-deoxyuracil and/or N4-ethyl-2'-deoxycytidine.

20. Method for targeted alteration of a duplex acceptor DNA sequence, comprising combining the duplex acceptor DNA sequence with a donor oligonucleotide, wherein the duplex acceptor DNA sequence contains a first DNA sequence and a second DNA sequence which is the complement of the first DNA sequence and wherein the donor oligonucleotide comprises a domain that comprises at least one mismatch with respect to the duplex acceptor DNA sequence to be altered, preferably with respect to the first DNA sequence, and wherein the oligonucleotide comprises a section that contains at least one modified nucleotide having a higher binding affinity compared to naturally occurring A, C, T or G and wherein the modified nucleotide binds stronger to a nucleotide in an opposite position in the first DNA sequence as compared to a naturally occurring nucleotide complementary to the nucleotide in an opposite position in the first DNA sequence, in the presence of proteins that are capable of targeted nucleotide exchange.

21. Method according to clause 20, wherein the modified nucleotide is defined in clauses 1-19.

22. Method according to clause 20, wherein the alteration is within a cell preferably selected from the group consisting of a plant cell, a fungal cell, a rodent cell, a primate cell, a human cell or a yeast cell.

23. Method according to clause 20, wherein the proteins are derived from a cell extract.

24. Method according to clause 23, wherein the cell extract is selected from the group consisting of a plant cell extract, a fungal cell extract, a rodent cell extract, a primate cell extract, a human cell extract or a yeast cell extract.

25. Method according to clause 20 or 21, wherein the alteration is a deletion, a substitution or an insertion of at least one nucleotide.

26. Method according to clause 21, wherein the cell is a eukaryotic cell, a plant cell, a non-human mammalian cell or a human cell.

27. Method according to any of the previous clauses, wherein the target DNA is from fungi, bacteria, plants, mammals or humans.

28. Method according to any of the previous clauses, wherein the duplex DNA is from genomic DNA, linear DNA, mammalian artificial chromosomes, bacterial artificial chromosomes, yeast artificial chromosomes, plant artificial chromosomes, nuclear chromosomal DNA, organelle chromosomal DNA, episomal DNA.

29. Method according to any of the previous clauses, for altering a cell, correcting a mutation by restoration to wild type, inducing a mutation, inactivating an enzyme by disruption of coding region, modifying bioactivity of an enzyme by altering coding region, modifying a protein by disrupting the coding region.

30. Use of an oligonucleotide as defined in clauses 1-19, for altering a cell, correcting a mutation by restoration to wild type, inducing a mutation, inactivating an enzyme by disruption of coding region, modifying bioactivity of an enzyme by altering coding region, modifying a protein by disrupting the coding region, mismatch repair, targeted alteration of (plant)genetic material, including gene mutation, targeted gene repair and gene knockout.

31. Use of an oligonucleotide for enhanced targeted alteration of a duplex DNA sequence, the duplex DNA sequence containing a first DNA sequence and a second DNA sequence which is the complement of the first DNA sequence, the oligonucleotide comprising a domain that is capable of hybridizing to the first DNA sequence, which domain comprises at least one mismatch with respect to the first DNA sequence, and wherein the oligonucleotide comprises a section that contains modified nucleotides, wherein the modified nucleotides have a higher binding affinity compared to naturally occurring A, C, T or G and wherein the modified nucleotides bind stronger to a nucleotide in an opposite position in the first DNA sequence as compared to a naturally occurring nucleotide complementary to the nucleotide in an opposite position in the first DNA sequence.

32. Kit comprising an oligonucleotide as defined in clauses 1-19.

33. Modified genetic material produced by the method of clauses 20-29.

34. Cell comprising the modified genetic material of clause 33.

35. Plant or plant part produced by the method of clauses 20-29 or comprising the genetic material of clause 33.

Example:

Targeted Nucleotide exchange of the ALS gene in Tobacco

**Tobacco shoot cultures**

The source material for this example is tobacco SR1 *in vitro* shoot cultures. They are grown under sterile conditions in large glass jars (750 ml capacity) in MS20 medium in growth chambers at a temperature of 25/20°C (day/night) and a photon flux density of 80 $\mu E.m^{-2}.s^{-1}$ with a photoperiod of 16/24 h. MS20 medium is basic Murashige and Skoog's medium (Murashige, T. and Skoog, F., Physiologia Plantarum, 15: 473-497, 1962) containing 2% (w/v) sucrose, no added hormones and 0.8% Difco agar. The shoots are subcultured every 3 weeks to fresh medium.

**Demethylation via azacytidine**

Two weeks prior to protoplast isolation, the tobacco in vitro shoot cultures can be subcultured on MS20 medium containing 75 μg/ml 5-azacytidine in order to demethylate the target locus DNA.

**Protoplast isolation**

For the isolation of mesophyll protoplasts, fully expanded leaves of 3-6 week old shoot culture plants are harvested. The leaves are carefully sliced, through the lower epidermis and from the midrib outward, into 1 mm thin strips. The sliced leaves are transferred to large (100 mm x 100 mm) Petri dishes containing 45 ml MDE basal medium for a preplasmolysis treatment of 30 min. MDE basal medium contained 0.25 g KCl, 1.0 g $MgSO_4.7H_2O$, 0.136 g of $KH_2PO_4$, 2.5 g polyvinylpyrrolidone (MW 10,000), 6 mg naphthalene acetic acid and 2 mg 6-benzylaminopurine in a total volume of 900 ml. The osmolality of the solution is adjusted to 600 $mOsm.kg^{-1}$ with sorbitol, the pH to 5.7.

After preplasmolysis, 5 ml of enzyme stock is added to each Petri dish. The enzyme stock consists of 750 mg Cellulase Onozuka R10, 500 mg driselase and 250 mg macerozyme R10 per 100 ml, filtered over Whatman paper and filter-sterilized. The Petri dishes are sealed and incubated overnight in the dark at 25°C without movement to digest the cell walls.

Next morning, the dishes are gently swirled to release the protoplasts. The protoplast suspension is passed through 500 μm and 100 μm sieves into 250 ml Erlenmeyer flasks, mixed with an equal volume of KCl wash medium, and centrifuged in 50 ml tubes at 85 x g for 10 min. KCl wash medium consisted of 2.0 g $CaCl_2.2H_2O$ per liter and a quantity of KCl to bring the osmolality to 540 $mOsm.kg^{-1}$.

The protoplasts, recovered from the centrifugation pellets, are resuspended in MLm wash medium and centrifuged once again in 10 ml glass tubes at 85 x g for 10 min. MLm wash medium contained the macro-nutrients of MS medium (ref) at half the normal concentration, 2.2 g of $CaCl_2.2H_2O$ per liter and a quantity of mannitol to bring the osmolality to 540 mOsm.kg$^{-1}$.

The protoplasts, recovered from the pellets of this second centrifugation step, are resuspended in MLs wash medium and centrifuged again in 10 ml glass tubes at 85-100 x g for 10 min. MLs wash medium contained the macronutrients of MS medium (ref) at half the normal concentration, 2.2 g of $CaCl_2.2H_2O$ per liter and a quantity of sucrose to bring the osmolality to 540 mOsm.kg$^{-1}$.

The protoplasts are recovered from the floating band and resuspended in an equal volume of KCl wash medium. Their densities are counted using a haemocytometer. Subsequently, the protoplasts are centrifuged again in 10 ml glass tubes at 85 x g for 5 min and the pellets resuspended at a density of 1 x $10^5$ protoplasts ml$^{-1}$ in electroporation medium. All solutions are kept sterile, and all manipulations are done under sterile conditions.

**ALS target gene and design of single stranded oligonucleotides**

In the tobacco acetolactate synthase (ALS) SurA gene (Gene Bank Accession X07644) the amino acid conversion P194Q makes the ALS protein insensitive to the sulfonylurea herbicide chlorsulfuron. The single stranded oligonucleotide NtALSP194Q can be used as a model to introduce this single nucleotide changes at the tobacco ALS gene by TNE.

NtALSP194Q

5'CAACAATAGGAGTTTCCTGAAAAGCATCAGTACCTATCATCCTACGTTGCACTTGACCTG TTATAGCAACAATGGGGAC 3' [SEQ ID NO: 1]

The mismatch nucleotide is underlined at position 48 from the 5' end.

**Protoplast electroporation**

Using PHBS as an electroporation medium (10mM Hepes, pH 7.2; 0.2 M mannitol, 150mM NaCL; 5 mM CaCL2) and with a density of protoplasts in the medium during electroporation of ca. 1x$10^6$ /ml, the electroporation settings are 250V (625 V cm$^{-1}$) charge and 800 μF capacitance with a recovery time between pulse and cultivation of 10 minutes. For each electroporation ca. 1-2 μg oligonucleotide are used and 20 μg plasmid per 800 microliter electroporation = 25 μg/ml.

**Protoplast regeneration and chlorsulfuron selection**

After the electroporation treatment, the protoplasts are placed on ice for 30 min to recover. They are then resuspended in To culture medium at a density of 1 x $10^5$ protoplasts ml$^{-1}$. To culture medium contained (per liter, pH 5.7) 950 mg $KNO_3$, 825 mg $NH_4NO_3$, 220 mg $CaCl_2.2H_2O$, 185 mg $MgSO_4.7H_2O$, 85 mg $KH_2PO_4$, 27.85 mg $FeSO_4.7H_2O$, 37.25 mg $Na_2EDTA.2H_2O$, the micro-nutrients according to Heller's medium (Heller, R., Ann Sci Nat Bot Biol veg 14: 1-223, 1953), vitamins according to Morel and Wetmore's medium (Morel, G. and R.H. Wetmore, Amer. J. Bot. 38: 138-40, 1951), 2% (w/v) sucrose, 3 mg naphthalene acetic acid, 1 mg 6-benzylaminopurine and a quantity of mannitol to bring the osmolality to 540 mOsm.kg$^{-1}$.

The protoplasts resuspended in To culture medium are then mixed with an equal volume of a solution of 1.6% SeaPlaque Low Melting Temperature Agarose in To culture medium, kept liquid after autoclaving in a waterbath at 30°C. After mixing, the suspension is gently pipetted in 2.5 ml aliquots into 5 cm Petri dishes. The dishes are sealed and incubated at 25/20°C (16/24 h photoperiod) in the dark.

After 8-10 days incubation in the dark, the agarose medium is cut into 6 equal pie-shaped parts, which are transferred to 10 cm Petri dishes each containing 22.5 ml of liquid $MAP_1AO$ medium. This medium consisted of (per liter, pH 5.7) 950 mg $KNO_3$, 825 mg $NH_4NO_3$, 220 mg $CaCl_2.2H_2O$, 185 mg $MgSO_4.7H_2O$, 85 mg $KH_2PO_4$, 27.85 mg $FeSO_4.7H_2O$, 37.25 mg $Na_2EDTA.2H_2O$, the micro-nutrients according to Murashige and Skoog's medium (Murashige, T. and Skoog, F., Physiologia Plantarum, 15: 473-497, 1962) at one tenth of the original concentration, vitamins according to Morel and Wetmore's medium (Morel, G. and R.H. Wetmore, Amer. J. Bot. 38: 138-40, 1951), 6 mg pyruvate, 12 mg each of malic acid, fumaric acid and citric acid, 3% (w/v) sucrose, 6% (w/v) mannitol, 0.03 mg naphthalene acetic acid and 0.1 mg 6-benzylaminopurine. For purposes of selection of colonies with a successful base conversion, 41 nM chlorsulfuron is also added to the medium. The Petri dishes are incubated at 25/20°C in low light (photon flux density of 20 μE.m$^{-2}$.s$^{-1}$) at a photoperiod of 16/24 h. After two weeks, the Petri dishes are transferred to full light (80 μE.m$^{-2}$.s$^{-1}$). During this period of selection, most protoplasts died. Only protoplasts, in which through the action of the oligonucleotides a base change has occurred in the target gene so as to confer resistance to the herbicide, divide and proliferate into protoplast-derived microcolonies.

Six to eight weeks after isolation, the protoplast-derived colonies are transferred to $MAP_1$ medium. The agarose beads by this time fall apart sufficiently to transfer the microcolonies with a wide-mouthed sterile pipette, or else they are individually transferred with forceps. $MAP_1$ medium has the same composition as $MAP_1AO$ medium, with however

3% (w/v) mannitol instead of 6%, and 46.2 mg.$l^{-1}$ histidine (pH 5.7). It was solidified with 0.8% (w/v) Difco agar.

After 2-3 weeks of growth on this solid medium, the colonies are transferred to regeneration medium RP, 50 colonies per 10 cm Petri dish. RP medium consisted of (per liter, pH 5.7) 273 mg $KNO_3$, 416 mg $Ca(NO_3)_2.4H_2O$, 392 mg $Mg(NO_3)_2.6H_2O$, 57 mg $MgSO_4.7H_2O$, 233 mg $(NH_4)_2SO_4$, 271 mg $KH_2PO_4$, 27.85 mg $FeSO_4.7H_2O$, 37.25 mg $Na_2EDTA.2H_2O$, the micro-nutrients according to Murashige and Skoog's medium (Murashige, T. and Skoog, F., Physiologia Plantarum, 15: 473-497, 1962) at one fifth of the published concentration, vitamins according to Morel and Wetmore's medium (Morel, G. and R.H. Wetmore, Amer. J. Bot. 38: 138-40, 1951), 0.05% (w/v) sucrose, 1.8% (w/v) mannitol, 0.25 mg zeatin and 41nM chlorsulfuron, and is solidified with 0.8% (w/v) Difco agar.

**PCR amplification of target gene**

DNA is isolated from butafenacil and chlorsulfuron resistant tobacco microcolonies using the DNeasy kit (Qiagen). Total tobacco DNA is then used as a template in the PCR reaction. Conversion of the targeted codon in the tobacco ALS gene is detected using the primers 5'GGTCAAGTGCCACGTAGGAT [SEQ ID NO: 2] & 5'GGGTGCTTCACTTTCTGCTC [SEQ ID NO: 3] that amplify a 776 bp fragment of this gene, including codon 194.

**Sequencing to proof nucleotide conversion**

Nucleotide conversion in the herbicide resistant tobacco callus is confirmed by sequencing the PCR products obtained from such callus. Conversion of the tobacco ALS P194 codon (CCA to CAA) results in a double peak at the second position of the codon (C/A).

The following single stranded oligos are tested for production of a point mutation in the tobacco ALS gene leading to herbicide resistance. The oligonucleotides contain modified nucleotides to increase the efficiency of targeted nucleotide exchange.

5'-CAACAATAGGAGTTTCCTGAAAAGCATCAGTACCTATCATCCTACGTTGCACTTGA CCTGTTATAGCAACAATGGGGAC-3' [SEQ ID NO: 4]

The above target oligonucleotide is 79 nucleotides in length. The mismatch in the oligonucleotide is underlined at position 48 from the 5'end. The rest of the oligonucleotide is identical to the ALS gene. Modified oligonucleotides are altered at the base or sugar (in which case the altered nucleotide in the sequence is indicated). Alternatively, the phosphate linkages linking nucleotides can also be altered. In this case an asterisk (*) is inserted between the nucleotides to indicate the position of such linkages./esp

<u>Oligonucleotide 1: Morpholino Phosphoroamidate</u>

C*A*A*C*A*A*T*A*G*G*A*G*T*T*T*C*C*T*G*A*A*A*A*G*C*A*T*C*A*G*T*
A*C*C*T*A*T*C*A*T*C*C*T*A*C*G*T*T*G*C*A*C*T*T*G*A*C*C*T*G*T*T*
A*T*A*G*C*A*A*C*A*A*T*G*G*G*G*A*C [SEQ ID NO:5]

Morpholino Phosphoroamidate linkages are modifications of the phosphate backbone. Since it is not yet possible to make DNA/_Morpholino Phosphoroamidate hybrids, all the phosphates in the oligo are modified in this embodiment.

<u>Oligonucleotide 2: 2'-fluoroarabinonucleic acid (lowercase letters)</u>
caACAATAGGAGTTTCCTGAAAAGCATCAGTACCTATCATCCTACGTTGCACTTGACCTGTT ATAGCAACAATGGGGac [SEQ ID NO: 6]

<u>Oligonucleotide 3: 6-chloro-2-methoxyacridine modified nucleotides (asterisk, *)</u>
C*AACAATAGGAGTTTCCTGAAAAGCATCAGTACCTATCATCCTACGTTGCACTTGACCTGT TATAGCAACAATGGGGAC* [SEQ ID NO: 7]
CAACAATAGGAGTTTCCTGAAAAGCATCAGTACCTATCATCCTACG*TTG*CACTTGACCTG TTATAGCAACAATGGGGAC [SEQ ID NO: 8]
CAACAATAGGAGTTTCCTGAAAAGCATCAGTACCTATCATCCTAC*G1TTG*C1ACTTGACC TGTTATAGCAACAATGGGGAC [SEQ ID NO: 9]
CAACAATAGGAGTTTCCTGAAAAGCATCAGTACCTATCATCCTAC*GTTGC*ACTTGACCTG TTATAGCAACAATGGGGAC [SEQ ID NO: 10]
C*AACAATAGGAGTTTCCTGAAAAGCATCAGTACCTATCATCCTACG*TTG*CACTTGACCT GTTATAGCAACAATGGGGAC* [SEQ ID NO: 11]
C*AACAATAGGAGTTTCCTGAAAAGCATCAGTACCTATCATCCTAC*G*TTG*C*ACTTGAC CTGTTATAGCAACAATGGGGAC* [SEQ ID NO: 12]

C*AACAATAGGAGTTTCCTGAAAAGCATCAGTACCTATCATCCTAC*GTTGC*ACTTGACCT GTTATAGCAACAATGGGGAC* [SEQ ID NO: 13]

<u>Oligonucleotide 4: Phosphorothioate linkages (asterisk, *)</u>
C*A*A*C*AATAGGAGTTTCCTGAAAAGCATCAGTACCTATCATCCTACGTTGCACTTGACC TGTTATAGCAACAATGG*G*G*A*C [SEQ ID NO: 14]

<u>Oligonucleotide 5: Methylphosphonate linkages (asterisk, *)</u>
C*A*A*C*A*A*T*A*G*G*A*G*T*T*T*C*C*T*G*A*A*A*A*G*C*A*T*C*A*G*T*
A*C*C*T*A*T*C*A*T*C*C*T*A*C*G*T*T*G*C*A*C*T*T*G*A*C*C*T*G*T*T*
A*T*A*G*C*A*A*C*A*A*T*G*G*G*G*A*C [SEQ ID NO: 15]

<u>Oligonucleotide 6: αL-LNA (lowercase letters)</u>
CaAcAaTaGgAgTtTcCtGaAaAgCaTcAgTaCcTaTcAtCcTaCgTtGcAcTtGaCcTgTt AtAgCaAcAaTgGgGaC [SEQ ID NO: 16]
cAaCaAtAgGaGtTtCcTgAaAaGcAtCaGtAcCtAtCaTcCtAcGtTgCaCtTgAcCtGtT aTaGcAaCaAtGgGgAc [SEQ ID NO: 17]

<u>Oligonucleotide 7: 2'-O-methylinosine (indicated by an I)</u>
IIACAATAGGAGTTTCCTGAAAAGCATCAGTACCTATCATCCTACGTTGCACTTGACCTGTT ATAGCAACAAT-GGGGII [SEQ ID NO: 18]
IIACAATAGGAGTTTCCTGAAAAGCATCAGTACCTATCATCCTACGITICACTTGACCTGTT ATAGCAACAATGGGGII [SEQ ID NO: 19]

<u>Oligonucleotide 8: 2'-fluoro-RNA (C and U) (only possible for C and U/T residues, represented as lowercase)</u>
cAACAATAGGAGTTTCCTGAAAAGCATCAGTACCTATCATCCTACGTTGCACTTGACCTGTT ATAGCAACAAT-GGGGAc [SEQ ID NO: 20]
cAACAATAGGAGTTTCCTGAAAAGCATCAGTACCTATCATCCTACGuTGcACTTGACCTGTT ATAGCAACAATGGGGAc [SEQ ID NO: 21]

<u>Oligonucleotide 9: 2'-O-methoxyethyl RNA (MOE) (RNA nucleotides represented as lowercase)</u>
caACAATAGGAGTTTCCTGAAAAGCATCAGTACCTATCATCCTACGTTGCACTTGACCTGTT ATAGCAACAAT-GGGGac [SEQ ID NO: 22]
CAACAATAGGAGTTTCCTGAAAAGCATCAGTACCTATCATCCTACGuTgCACTTGACCTGTT ATAGCAACAATGGGGAC [SEQ ID NO: 23]
caACAATAGGAGTTTCCTGAAAAGCATCAGTACCTATCATCCTACGuTgCACTTGACCTGTT ATAGCAACAATGGGGac [SEQ ID NO: 24]

<u>Oligonucleotide 10: $N^3$-$P^5$-phosphoroamidate linkages (asterisk, *)</u>
C*A*A*C*A*A*T*A*G*G*A*G*T*T*T*C*C*T*G*A*A*A*A*G*C*A*T*C*A*G*T*
A*C*C*T*A*T*C*A*T*C*C*T*A*C*G*T*T*G*C*A*C*T*T*G*A*C*C*T*G*T*T*
A*T*A*G*C*A*A*C*A*A*T*G*G*G*G*A*C [SEQ ID NO: 25]

<u>Oligonucleotide 11: 2-amino-adenosine (lowercase)</u>
CAACAATAGGAGTTTCCTGAAAAGCATCAGTACCTATCATCCTaCGTTGCaCTTGACCTGTT ATAGCAACAAT-GGGGAC [SEQ ID NO: 26]

<u>Oligonucleotide 12: Super A (lowercase)</u>
CaaCaaTaGGaGTTTCCTGaaaaGCaTCaGTaCCTaTCaTCCTaCGTTGCaCTTGaCCTGTT aTaGCaaCaaT-GGGGaC [SEQ ID NO: 27]

<u>Oligonucleotide 13: Super T (lowercase)</u>
CAACAAtAGGAGtttCCtGAAAAGCAtCAGtACCtAtCAtCCtACGtTGCACttGACCtGtt AtAGCAACAAtGGGGAC [SEQ ID NO: 28]

<u>Oligonucleotide 14: 5-methyl-isodeoxycytidine (lowercase)</u>
CAACAATAGGAGTTTCCTGAAAAGCATCAGTACCTATCATCcTAcGTTGcAcTTGACCTGTT ATAGCAACAAT-GGGGAC [SEQ ID NO: 29]

Oligonucleotide 15: 5-propynyl-2-deoxynucleoside (lowercase)

CAACAATAGGAGTTTCCTGAAAAGCATCAGTACCTATCATCCTACGtTgCACTTGACCTGTT ATAGCAACAATGGGGAC [SEQ ID NO: 30]

CAACAATAGGAGTTTCCTGAAAAGCATCAGTACCTATCATCCTACgTTGcACTTGACCTGTT ATAGCAACAATGGGGAC [SEQ ID NO: 31]

CAACAATAGGAGTTTCCTGAAAAGCATCAGTACCTATCATCCTACgtTgcACTTGACCTGTT ATAGCAACAATGGGGAC [SEQ ID NO: 32]

Oligonucleotide 16: $N^4$-ethyl-2'-deoxycytidine (lowercase)

CAACAATAGGAGTTTCCTGAAAAGCATCAGTACCTATCATCCTACGtTgCACTTGACCTGTT ATAGCAACAATGGGGAC [SEQ ID NO: 33]

CAACAATAGGAGTTTCCTGAAAAGCATCAGTACCTATCATCCTACgTTGcACTTGACCTGTT ATAGCAACAATGGGGAC [SEQ ID NO: 34]

CAACAATAGGAGTTTCCTGAAAAGCATCAGTACCTATCATCCTACgtTgcACTTGACCTGTT ATAGCAACAATGGGGAC [SEQ ID NO: 35]

The sequences used in this application are listed in the Table below, together with the position of their modification, counted from the 5'-end. Backbone modifications between x and x+1 are positioned at x (between position 4 and 5 is indicated as 4)

Table

| SEQ ID NO | Sequence 5'- 3' | Modification positions |
|---|---|---|
| 1 | CAACAATAGGAGTTTCCTGAAAAGCATCAGTA CCTATCATCCTACGTTGCACTTGACCTGTTAT AGCAACAATGGGGAC | None, target |
| 2 | GGTCAAGTGCCACGTAGGAT | None, Primer |
| 3 | GGGTGCTTCACTTTCTGCTC | None, Primer |
| 4 | CAACAATAGGAGTTTCCTGAAAAGCATCAGTA CCTATCATCCTACGTTGCACTTGACCTGTTAT AGCAACAATGGGGAC | None, target |
| 5 | C*A*A*C*A*A*T*A*G*G*A*G*T*T*T*C* C*T*G*A*A*A*A*G*C*A*T*C*A*G*T*A* C*C*T*A*T*C*A*T*C*C*T*A*C*G*T*T* G*C*A*C*T*T*G*A*C*C*T*G*T*T*A*T* A*G*C*A*A*C*A*A*T*G*G*G*G*A*C | Backbone 1-79 |
| 6 | caACAATAGGAGTTTCCTGAAAAGCATCAGTA CCTATCATCCTACGTTGCACTTGACCTGTTAT AGCAACAATGGGGac | 1, 2, 78, 79 |

(continued)

| SEQ ID NO | Sequence 5'- 3' | Modification positions |
|---|---|---|
| 7 | C*AACAATAGGAGTTTCCTGAAAAGCATCAGTACCTATCATCCTACGTTGCACTTGACCTGTTATAGCAACAATGGGGAC* | 1,79 |
| 8 | CAACAATAGGAGTTTCCTGAAAAGCATCAGTACCTATCATCCTACG*TTG*CACTTGACCTGTTATAGCAACAATGGGGAC | 46,49 |
| 9 | CAACAATAGGAGTTTCCTGAAAAGCATCAGTACCTATCATCCTAC*G*TTG*C*ACTTGACCTGTTATAGCAACAATGGGGAC | 46, 47, 49, 50 |
| 10 | CAACAATAGGAGTTTCCTGAAAAGCATCAGTACCTATCATCCTAC*GTTGC*ACTTGACCTGTTATAGCAACAATGGGGAC | 45,50 |
| 11 | C*AACAATAGGAGTTTCCTGAAAAGCATCAGTACCTATCATCCTACG*TTG*CACTTGACCTGTTATAGCAACAATGGGGAC* | 1, 46, 49, 79 |
| 12 | C*AACAATAGGAGTTTCCTGAAAAGCATCAGTACCTATCATCCTAC*G*TTG*C*ACTTGACCTGTTATAGCAACAATGGGGAC* | 1, 45, 46, 49, 50, 79 |
| 13 | C*AACAATAGGAGTTTCCTGAAAAGCATCAGTACCTATCATCCTAC*GTTGC*ACTTGACCTGTTATAGCAACAATGGGGAC* | 1, 45, 50,79 |
| 14 | C*A*A*C*AATAGGAGTTTCCTGAAAAGCATCAGTACCTATCATCCTACGTTGCACTTGACCTGTTATAGCAACAATGG*G*G*A*C | 1, 2, 3, 4, 75, 76, 77,78 |

(continued)

| SEQ ID NO | Sequence 5'- 3' | Modification positions |
|---|---|---|
| 15 | C*A*A*C*A*A*T*A*G*G*A*G*T*T*T*C*<br>C*T*G*A*A*A*A*G*C*A*T*C*A*G*T*A*<br>C*C*T*A*T*C*A*T*C*C*T*A*C*G*T*T*<br>G*C*A*C*T*T*G*A*C*C*T*G*T*T*A*T*<br>A*G*C*A*A*C*A*A*T*G*G*G*A*C | Backbone 1-79 |
| 16 | CaAcAaTaGgAgTtTcCtGaAaAgCaTcAgTa<br>CcTaTcAtCcTaCgTtGcAcTtGaCcTgTtAt<br>AgCaAcAaTgGgGaC | 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78 |
| 17 | cAaCaAtAgGaGtTtCcTgAaAaGcAtCaGtA<br>cCtAtCaTcCtAcGtTgCaCtTgAcCtGtTaT | 1, 3, 5, 7, 9, 11, 13, 15, |
| | aGcAaCaAtGgGgAc | 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79 |
| 18 | IIACAATAGGAGTTTCCTGAAAAGCATCAGTA<br>CCTATCATCCTACGTTGCACTTGACCTGTTAT<br>AGCAACAATGGGGII | 1, 2, 78, 79 |
| 19 | IIACAATAGGAGTTTCCTGAAAAGCATCAGTA<br>CCTATCATCCTACGITICACTTGACCTGTTAT<br>AGCAACAATGGGGII | 1, 2, 47, 49, 78, 79 |
| 20 | cAACAATAGGAGTTTCCTGAAAAGCATCAGTA<br>CCTATCATCCTACGTTGCACTTGACCTGTTAT<br>AGCAACAATGGGGAc | 1, 79 |
| 21 | cAACAATAGGAGTTTCCTGAAAAGCATCAGTA<br>CCTATCATCCTACGuTGcACTTGACCTGTTAT<br>AGCAACAATGGGGAc | 1, 47, 50, 79 |
| 22 | caACAATAGGAGTTTCCTGAAAAGCATCAGTA<br>CCTATCATCCTACGTTGCACTTGACCTGTTAT<br>AGCAACAATGGGGac | 1, 2, 78, 79 |

(continued)

| SEQ ID NO | Sequence 5'- 3' | Modification positions |
|---|---|---|
| 23 | CAACAATAGGAGTTTCCTGAAAAGCATCAGTACCTATCATCCTACGuTgCACTTGACCTGTTATAGCAACAATGGGGAC | 47, 49 |
| 24 | caACAATAGGAGTTTCCTGAAAAGCATCAGTACCTATCATCCTACGuTgCACTTGACCTGTTATAGCAACAATGGGGac | 1, 2, 47, 49, 78, 79 |
| 25 | C*A*A*C*A*A*T*A*G*G*A*G*T*T*T*C*C*T*G*A*A*A*A*G*C*A*T*C*A*G*T*A*C*C*T*A*T*C*A*T*C*C*T*A*C*G*T*T*G*C*A*C*T*T*G*A*C*C*T*G*T*T*A*T*A*G*C*A*A*C*A*A*T*G*G*G*G*A*C | Backbone 1-79 |
| 26 | CAACAATAGGAGTTTCCTGAAAAGCATCAGTACCTATCATCCTaCGTTGCaCTTGACCTGTTATAGCAACAATGGGGAC | 44, 51 |
| 27 | CaaCaaTaGGaGTTTCCTGaaaaGCaTCaGTaCCTaTCaTCCTaCGTTGCaCTTGaCCTGTTaTaGCaaCaaTGGGGaC | 2, 3, 5, 6, 8, 11, 20-23, 26, 29, 32, 36, 39, 44, 51, 56, 63, 65, 68, 69, 71, 72, 78 |
| 28 | CAACAAtAGGAGtttCCtGAAAAGCAtCAGtACCtAtCAtCCtACGtTGCACttGACCtGttAtAGCAACAAtGGGGAC | 7, 13, 14, 15, 18, 27, 31, 35, 37, 40, 43, 47, 53, 54, 59, 61, 62, 64, 73 |
| 29 | CAACAATAGGAGTTTCCTGAAAAGCATCAGTACCTATCATCcTAcGTTGcAcTTGACCTGTTATAGCAACAATGGGGAC | 42, 45, 50, 52 |
| 30 | CAACAATAGGAGTTTCCTGAAAAGCATCAGTACCTATCATCCTACGtTgCACTTGACCTGTTATAGCAACAATGGGGAC | 47, 49 |

(continued)

| SEQ ID NO | Sequence 5'- 3' | Modification positions |
|---|---|---|
| 31 | CAACAATAGGAGTTTCCTGAAAAGCATCAGTA CCTATCATCCTACgTTGcACTTGACCTGTTAT AGCAACAATGGGGAC | 46, 50 |
| 32 | CAACAATAGGAGTTTCCTGAAAAGCATCAGTA CCTATCATCCTACgtTgcACTTGACCTGTTAT | 46, 47, 49, 50 |
| 33 | AGCAACAATGGGGAC CAACAATAGGAGTTTCCTGAAAAGCATCAGTA CCTATCATCCTACGtTgCACTTGACCTGTTAT AGCAACAATGGGGAC | 47, 49 |
| 34 | CAACAATAGGAGTTTCCTGAAAAGCATCAGTA CCTATCATCCTACgTTGcACTTGACCTGTTAT AGCAACAATGGGGAC | 46, 50 |
| 35 | CAACAATAGGAGTTTCCTGAAAAGCATCAGTA CCTATCATCCTACgtTgcACTTGACCTGTTAT AGCAACAATGGGGAC | 46, 47, 49, 50 |

Example A:

The various oligonucleotides designs were tested using the cell free system as described herein elsewhere. Essentially, the cell free system is a TNE reaction in a test tube. Such a reaction has 3 components, (1) a plasmid containing a functional carbenicillin resistance gene and a defective kanamycin resistance gene. A point mutation (TAT to TAG) at codon 22 in the kanamycin gene (NPTII) producing a stop codon. (2) total protein extract from Arabidopsis containing all the proteins necessary for TNE and (3), an oligonucleotide carrying a mismatch designed to convert the G of the kanamycin stop codon into a C and thus restoring the kanamycin gene activity. These 3 components are mixed, incubated for 1 hour, the plasmid DNA is isolated and then electroporated to E. coli. The number of kanamycin resistant colonies is a measure of the TNE activity of a given oligonucleotide. The number of carbenicillin resistant colonies is used to calculate the electroporation efficiency.

In the table below the oligonucleotides tested for their TNE activity using the cell free system are listed. In each experiment an oligonucleotide made up of normal DNA is used. The TNE activity of a modified oligonucleotide is expressed as a fold change compared to the normal DNA oligo which is arbitrarily set at 1. Thus, a fold change below 1 means that the modified oligonucleotide works worse than the normal DNA oligo in our assay whereas a fold change above 1 indicates an improved TNE activity of the oligo.

| SEQ ID | Sequence, 5'-3' | Nucleotide modification (position) | Fold change |
|---|---|---|---|
| 36 | TGTGCCCAGTCGTAGCCGAATAGC | None | 1 |
| 37 | **T**GTGCCCAGTCGTAGCCGAATAG**C** | LNA (1, 24) | 1 |
| 38 | **TG**TGCCCAGTCGTAGCCGAATA**GC** | LNA (1, 2, 23, 24) | 0.4 |

(continued)

| SEQ ID | Sequence, 5'-3' | Nucleotide modification (position) | Fold change |
|---|---|---|---|
| 39 | **TG**TGCCCAGTCGTAGCCGAAT**AGC** | LNA (1-3, 22-24) | 0.16 |
| 40 | **TGTG**CCCAGTCGTAGCCGAA**TAGC** | LNA (1-4, 21-24) | <0.2 |
| 41 | TGTGCCCAG**T**CGT**A**GCCGAATAGC | LNA (10, 14) | 8 |
| 42 | TGTGCCCA**G**TCGTA**G**CCGAATAGC | LNA (9, 15) | 0.57 |
| 43 | TGTGCCC**A**GTCGTAG**C**CGAATAGC | LNA (8, 16) | 0.73 |
| 44 | TGTGCCC**A**G**T**CGT**A**G**C**CGAATAGC | LNA (8, 10, 14, 16) | 0.2 |
| 45 | TGTGCCCAGT**C**GT**A**GCCGAATAGC | LNA (11, 13) | 0.8 |
| 46 | TGTGCCCAGT**C**GTAG**C**CGAATAGC | N4-EDC (11, 16) | 7.7 |
| 47 | TGTGCCCAGT**C**G**U**AGCCGAATAGC | 5PC, 5PU (11, 13) | 6 |

The nucleotides in bold represent the positions of the modified nucleotides. LNA = locked nucleic acid, 5PC = 5-propanyl-2-deoxycytidine, 5PU = 5-propanyl-2-deoxyuracil, N4-EDC= N4-ethyl-deoxycytidine.

All oligos tested produce a G:G mismatch with the plasmid.

## Claims

1. Method for targeted alteration of a duplex acceptor DNA sequence, comprising combining the duplex acceptor DNA sequence with a donor oligonucleotide, wherein the duplex acceptor DNA sequence contains a first DNA sequence and a second DNA sequence which is the complement of the first DNA sequence and wherein the donor oligonucleotide comprises a domain that comprises at least one mismatch with respect to the duplex acceptor DNA sequence to be altered, preferably with respect to the first DNA sequence, and wherein the oligonucleotide comprises a section that contains at least one modified nucleotide having a higher binding affinity compared to naturally occurring A, C, T or G and wherein the modified nucleotide binds stronger to a nucleotide in an opposite position in the first DNA sequence as compared to a naturally occurring nucleotide complementary to the nucleotide in an opposite position in the first DNA sequence, in the presence of proteins that are capable of targeted nucleotide exchange, and wherein the at least one modified nucleotide is located adjacent to the mismatch, or within 2, 3, 4, 6, 7, 8, 9, or 10 nucleotides of the mismatch, preferably wherein the at least one modified nucleotide is located adjacent to the mismatch, or within 2, 3, or 4 nucleotides of the mismatch.

2. Method according to claim 1, wherein the oligonucleotide comprises at least 2 sections that independently contain at least one modified nucleotide.

3. Method according to any of claims 1-2, wherein the at least one modified nucleotide is selected from the group consisting of backbone modified nucleotides and/or base modified nucleotides.

4. Method according to any of the claims 1-3, wherein the oligonucleotide comprises nuclease resistant nucleotides.

5. Method according to any of the claims 1-4 wherein the modified nucleotide is selected form the group consisting of locked nucleic acids (LNA's), 5-propynylated nucleotides and/or N4-ethyl-2'-deoxycytidine.

6. Method according to any of claims 4 -5, wherein the nuclease resistant nucleotides are LNAs, preferably wherein the oligonucleotide comprises one or more LNA residues located at a distance of at least one base pair from the mismatch, even more preferably wherein one LNA is located at each side of the mismatch at a position of at least one base pair from the mismatch.

**7.** Method according to any of claims 1-6, wherein the oligonucleotide comprises one or more 5-propynylated nucleotides and or N4-ethyl-2'-deoxycytidine located adjacent to or at a distance of at least one base pair from the mismatch, preferably wherein one 5-propynylated nucleotide or N4-ethyl-2'-deoxycytidine is located at each side of the mismatch adjacent to or at a position of at least one base pair from the mismatch.

**8.** Method according to claim 7, wherein the 5-propynylated nucleotide are 5-propynyl-2-deoxycytidine and/or 5-propynyl-2-deoxyuracil and/or N4-ethyl-2'-deoxycytidine.

**9.** Method according to claim 4, wherein the nuclease resistant nucleotides are phosphorothioate modified nucleotides such that the oligonucleotide comprises at least one, two or preferably at least three phosphorothioate linkages in the oligonucleotide or wherein the nuclease resistant nucleotides are 2'-O methyl-substituted nucleotides.

**10.** Method according to any of the claims 1 -9, wherein the alteration is within a cell preferably selected from the group consisting of a eukaryotic cell, a plant cell, a fungal cell, a rodent cell, a primate cell, a human cell, a non-human mammalian cell or a yeast cell and wherein the alteration is a deletion, a substitution or an insertion of at least one nucleotide.

**11.** Method according to any of the previous claims, wherein the target DNA is from fungi, bacteria, plants, mammals or humans and/or wherein the duplex DNA is from genomic DNA, linear DNA, mammalian artificial chromosomes, bacterial artificial chromosomes, yeast artificial chromosomes, plant artificial chromosomes, nuclear chromosomal DNA, organelle chromosomal DNA, episomal DNA.

**12.** Method according to any of the previous claims, for altering a cell, correcting a mutation by restoration to wild type, inducing a mutation, inactivating an enzyme by disruption of coding region, modifying bioactivity of an enzyme by altering coding region, modifying a protein by disrupting the coding region.

**13.** Oligonucleotide for targeted alteration of a duplex DNA sequence, the duplex DNA sequence containing a first DNA sequence and a second DNA sequence which is the complement of the first DNA sequence, the oligonucleotide comprising a domain that is capable of hybridizing to the first DNA sequence, which domain comprises at least one mismatch with respect to the first DNA sequence, and wherein the oligonucleotide comprises at least one section that contains at least one modified nucleotide having a higher binding affinity compared to naturally occurring A, C, T or G and wherein the at least one modified nucleotide binds stronger to a nucleotide in an opposite position in the first DNA sequence as compared to a naturally occurring nucleotide complementary to the nucleotide in the opposite position in the first DNA sequence, and wherein the at least one modified nucleotide is located adjacent to the mismatch, or within 2, 3, 4, 6, 7, 8, 9, or 10 nucleotides of the mismatch, preferably wherein the at least one modified nucleotide is located adjacent to the mismatch, or within 2, 3, or 4 nucleotides of the mismatch.

**14.** Oligonucleotide according to claim 13 as described in any of the claims 1-9.

**15.** Use of an oligonucleotide for enhanced targeted alteration of a duplex DNA sequence, the duplex DNA sequence containing a first DNA sequence and a second DNA sequence which is the complement of the first DNA sequence, the oligonucleotide comprising a domain that is capable of hybridizing to the first DNA sequence, which domain comprises at least one mismatch with respect to the first DNA sequence, and wherein the oligonucleotide comprises a section that contains modified nucleotides, wherein the modified nucleotides have a higher binding affinity compared to naturally occurring A, C, T or G and wherein the modified nucleotides bind stronger to a nucleotide in an opposite position in the first DNA sequence as compared to a naturally occurring nucleotide complementary to the nucleotide in an opposite position in the first DNA sequence, and wherein the at least one modified nucleotide is located adjacent to the mismatch, or within 2, 3, 4, 6, 7, 8, 9, or 10 nucleotides of the mismatch, preferably wherein the at least one modified nucleotide is located adjacent to the mismatch, or within 2, 3, or 4 nucleotides of the mismatch.

**16.** Kit comprising an oligonucleotide as described in claims 1-9 or according to claims 13 -14.

**17.** Modified genetic material produced by the method of any of claims 1 - 12 or a cell comprising the modified genetic material.

**18.** Plant or plant part produced by the method of any of claims 1 - 12 or comprising the genetic material of claim 17.

Fig 1

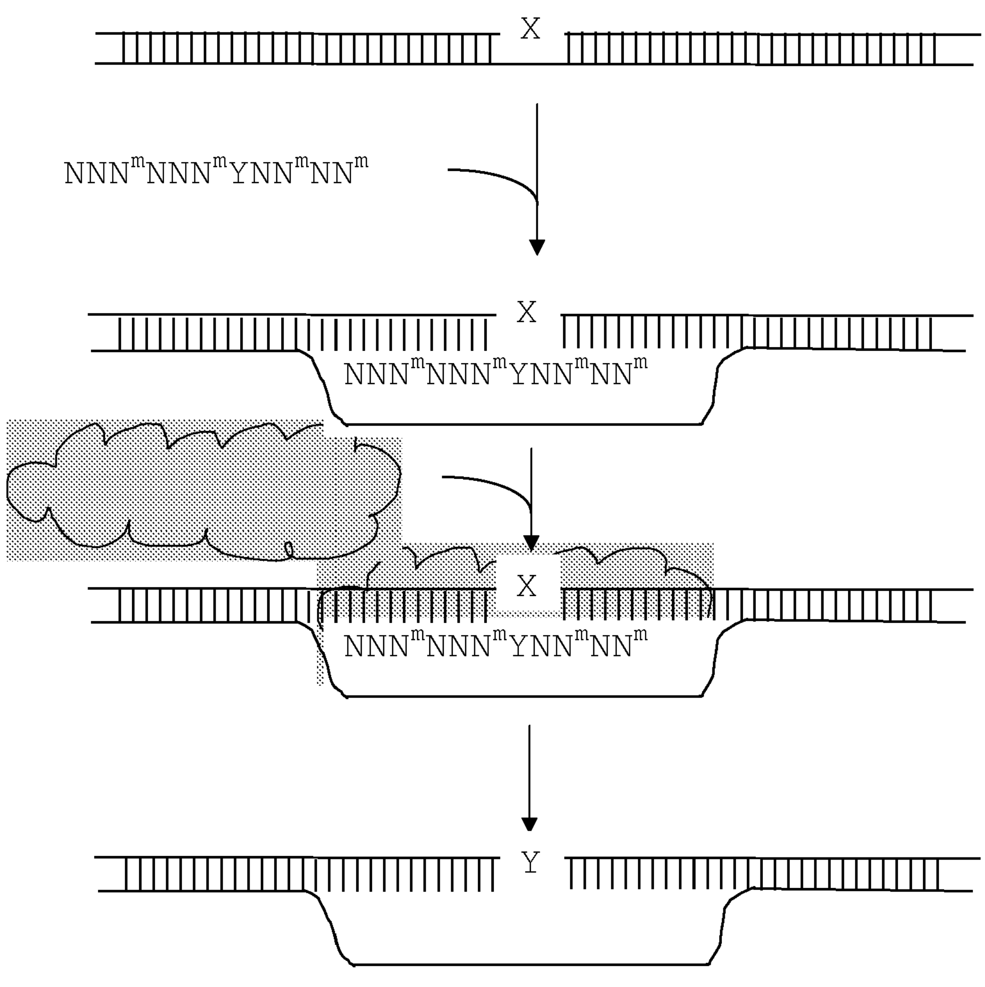

# Fig 2

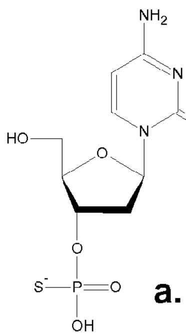

a.

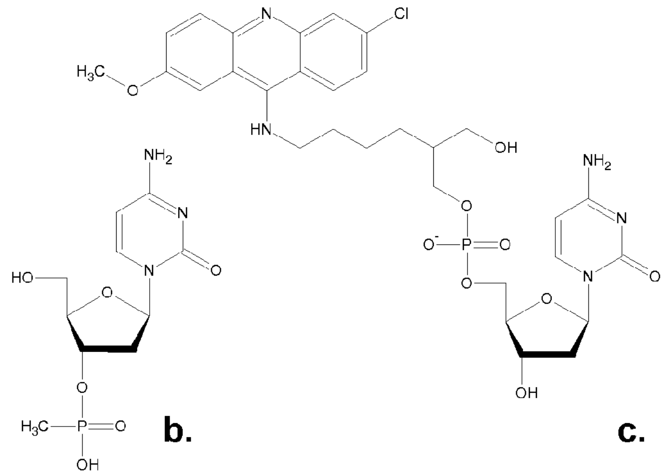

b.

c.

# Fig 3

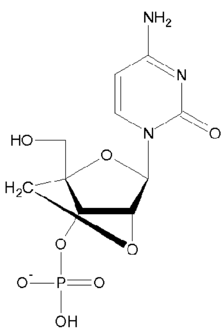

ß-D-oxy-LNA

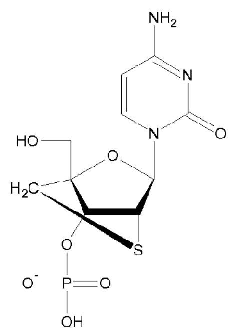

ß-D-thio-LNA

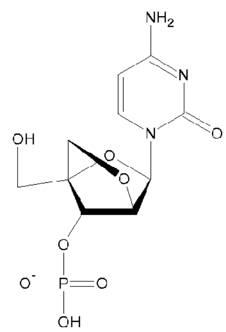

α-L-oxy-LNA

## Fig 4

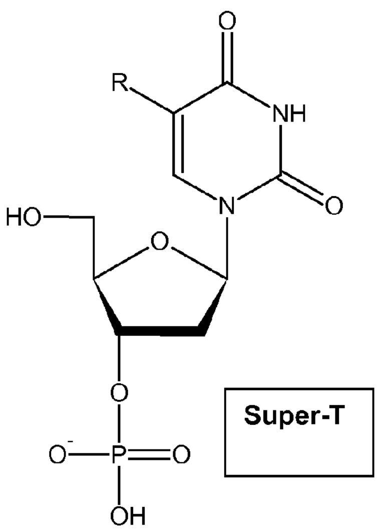

Super-T

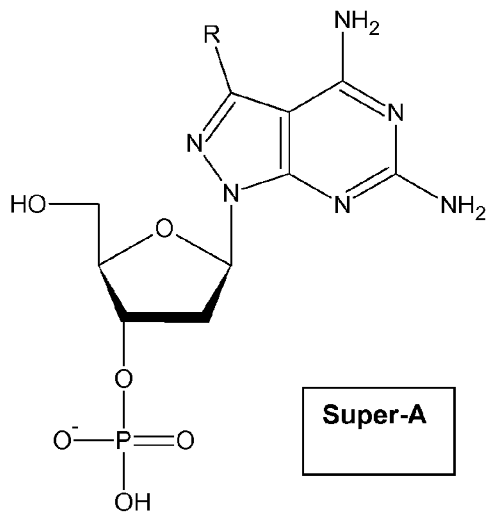

Super-A

## Fig 5

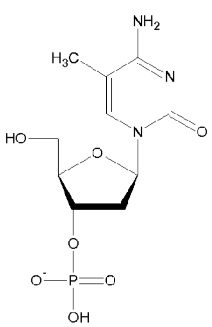

a.

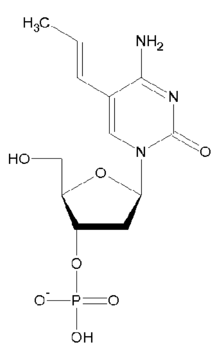

b.

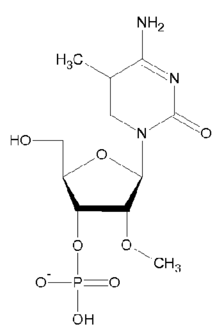

c.

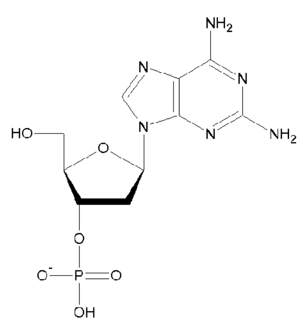

d.

Fig 6

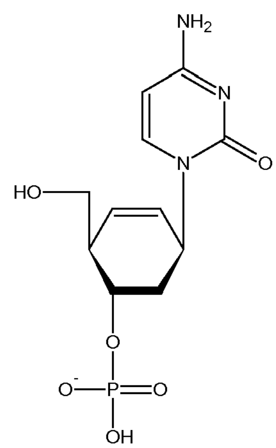

Europäisches Patentamt
European Patent Office
Office européen des brevets

# EUROPEAN SEARCH REPORT

Application Number
EP 10 18 7058

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/92512 A2 (UNIV DELAWARE [US]; KMIEC ERIC B [US]; GAMPER HOWARD B [US]; RICE MICH) 6 December 2001 (2001-12-06)<br>* claims 1,10-16 *<br>* page 4, paragraph 4 *<br>* page 5, paragraph 1 *<br>* page 6, last paragraph *<br>* page 9, paragraph 2 *<br>* page 13, paragraph 2 *<br>----- | 1-6,9-18 | INV.<br>C12Q1/68<br>C12N15/10 |
| X | PAREKH-OLMEDO HETAL ET AL: "Targeted nucleotide exchange in Saccharomyces cerevisiae directed by short oligonucleotides containing locked nucleic acids",<br>CHEMISTRY AND BIOLOGY, CURRENT BIOLOGY, LONDON, GB,<br>vol. 9, no. 10,<br>1 October 2002 (2002-10-01), pages 1073-1084, XP002424170,<br>ISSN: 1074-5521, DOI: DOI:10.1016/S1074-5521(02)00236-3 | 1-6,<br>10-18 | |
| Y | * the whole document *<br>----- | 7,8 | |
| X | LATORRA D ET AL: "Design considerations and effects of LNA in PCR primers",<br>MOLECULAR AND CELLULAR PROBES, ACADEMIC PRESS, LONDON, GB,<br>vol. 17, no. 5,<br>1 October 2003 (2003-10-01), pages 253-259, XP004466447,<br>ISSN: 0890-8508, DOI: DOI:10.1016/S0890-8508(03)00062-8<br>* the whole document *<br>-----<br>-/-- | 13,14,16 | |

TECHNICAL FIELDS SEARCHED (IPC)
C12Q
C12N

The present search report has been drawn up for all claims

3

| Place of search | Date of completion of the search | Examiner |
|---|---|---|
| Munich | 1 March 2011 | Persson, Anna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 18 7058

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | FRIEDEN M ET AL: "Expanding the design horizon of antisense oligonucleotides with alpha-L-LNA", NUCLEIC ACIDS RESEARCH, OXFORD UNIVERSITY PRESS, SURREY, GB, vol. 31, no. 21, 1 November 2003 (2003-11-01), pages 6365-6372, XP002281376, ISSN: 0305-1048, DOI: DOI:10.1093/NAR/GKG820 * the whole document * ----- | 13,14,16 | |
| X | BUSUTTIL S J ET AL: "Antisense Suppression of Protein Kinase C-Alpha and Delta in Vascular Smooth Muscle", JOURNAL OF SURGICAL RESEARCH, ACADEMIC PRESS INC., SAN DIEGO, CA, US, vol. 63, no. 1, 1 June 1996 (1996-06-01), pages 137-142, XP008081231, ISSN: 0022-4804, DOI: DOI:10.1006/JSRE.1996.0236 | 13,14,16 | |
| Y | * the whole document * ----- | 7,8 | TECHNICAL FIELDS SEARCHED (IPC) |
| X | NGUYEN HONG-KHANH ET AL: "Minimising the secondary structure of DNA targets by incorporation of a modified deoxynucleoside: Implications for nucleic acid analysis by hybridisation", NUCLEIC ACIDS RESEARCH, OXFORD UNIVERSITY PRESS, SURREY, GB, vol. 28, no. 20, 15 October 2000 (2000-10-15), pages 3904-3909, XP002248028, ISSN: 0305-1048, DOI: DOI:10.1093/NAR/28.20.3904 * the whole document * ----- | 13,14,16 | |

The present search report has been drawn up for all claims

3

| Place of search | Date of completion of the search | Examiner |
|---|---|---|
| Munich | 1 March 2011 | Persson, Anna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 18 7058

This annex lists the patent family members relating to the patent documents cited in the above-mentioned European search report.
The members are as contained in the European Patent Office EDP file on
The European Patent Office is in no way liable for these particulars which are merely given for the purpose of information.

01-03-2011

| Patent document cited in search report | Publication date | Patent family member(s) | Publication date |
|---|---|---|---|
| WO 0192512 A2 | 06-12-2001 | AU 6527701 A | 11-12-2001 |
| | | AU 2001265277 B2 | 07-09-2006 |
| | | CA 2410523 A1 | 06-12-2001 |
| | | EP 1297122 A2 | 02-04-2003 |
| | | US 2003236208 A1 | 25-12-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0173002 A
- WO 03027265 A
- WO 0187914 A
- WO 9958702 A
- WO 9748714 A
- WO 0210364 A
- WO 0192512 A
- WO 9220702 A
- WO 9220703 A
- WO 9312129 A
- US 5539082 A
- WO 9914226 A
- WO 0056748 A
- WO 0066604 A
- WO 9839352 A
- US 6043060 A
- US 6268490 B


### Non-patent literature cited in the description


- **Puchta.** *Plant Mol.Biol.,* 2002, vol. 48, 173
- *J. Exp. Botany,* 2005, vol. 56, 1
- **Alexeev ; Yoon.** *Nature Biotechnol.,* 1998, vol. 16, 1343
- **Rice.** *Nature Biotechnol.,* 2001, vol. 19, 321
- **Kmiec.** *J. Clin. Invest.,* 2003, vol. 112, 632
- **Beetham et al.** *PNAS,* 1999, vol. 96, 8774
- **Kochevenko ; Willmitzer.** *Plant Physiol.,* 2003, vol. 132, 174
- **Rice et al.** *Nat.Biotech.,* 2001, vol. 19, 321-326
- **Alexeev et al.** *Nature Biotech,* 2000, vol. 18, 43
- **Beetham et al.** *Proc.Natl.Acad.Sci.USA,* 1999, vol. 96, 8774-8778
- **Kochevenko et al.** *Plant Phys.,* 2003, vol. 132, 174-184
- **Okuzaki et al.** *Plant Cell Rep.,* 2004, vol. 22, 509-512
- **Zhu et al.** *PNAS,* 1999, vol. 96, 8768
- **Rice et al.** *Mol. Microbiol.,* 2001, vol. 40, 857-868
- **Liu et al.** *Nucl. Acids Res.,* 2002, vol. 30, 2742-2750
- **Parekh-Olmedo et al.** *Gene Therapy,* 2005, vol. 12, 639-646
- **Brachman et al.** *DNA Rep. (Amst),* 2005, vol. 4, 445-457
- **Stojic et al.** *DNA Repair (Amst),* 2004, vol. 3, 1091-1101
- **Zhu et al.** *Nature Biotech.,* 2000, vol. 18, 555-558
- **Fedier ; Fink.** *2004 Int. J Oncol.,* 2004, vol. 24 (4), 1039-47
- **Jiricny.** *Mutat Res.,* 1998, vol. 409 (3), 107-21
- **Kolodner ; Marsischky.** *Mol Cell.,* 1999, vol. 4 (3), 439-444
- *J. Biol. Chem.,* 1999, vol. 274 (38), 26668-26682
- *Curr. Opin. Genet. Dev.,* 1999, vol. 9 (1), 89-96
- **Fedier ; Fink.** *2004 Int J Oncol.,* 2004, vol. 24 (4), 1039-47
- **Peltomaki.** *J Clin Oncol.,* 2003, vol. 21 (6), 1174-9
- **Jiricny ; Nystrom-Lahti.** *Curr Opin Genet Dev.,* 2000, vol. 10 (2), 157-61
- **Bellacosa.** *J Cell Physiol.,* 2001, vol. 187 (2), 137-44
- **Aquilina ; Bignami.** *J Cell Physiol.,* May 2001, vol. 187 (2), 145-54
- **Baitinger et al.** *J Biol Chem.,* 05 December 2003, vol. 278 (49), 49505-11
- **Guarne et al.** *EMBO J.,* 27 October 2004, vol. 23 (21), 4134-45
- **Giron-Monzon et al.** *Biol Chem.,* 19 November 2004, vol. 279 (47), 49338-45
- **Joseph et al.** *DNA Repair (Amst),* 02 December 2004, vol. 3 (12), 1561-77
- **Drummond ; Bellacosa.** *Nucleic Acids Res.,* 01 June 2001, vol. 29 (11), 2234-43
- **Alberts et al.** Molecular Biology of the Cell. Garland publishing, 1989, 234ff
- **Patel.** *Nature,* 1993, vol. 365, 490
- **Nielsen et al.** *Science,* 1991, vol. 254, 1497
- **Egholm.** *J. Am. Chem. Soc.,* 1992, vol. 114, 1895
- **Knudson et al.** *Nucleic Acids Research,* 1996, vol. 24, 494
- **Nielsen et al.** *J. Am. Chem. Soc.,* 1996, vol. 118, 2287

- **Egholm et al.** *Science,* 1991, vol. 254, 1497
- **Egholm et al.** *J. Am. Chem. Soc.,* 1992, vol. 114, 1895
- **Egholm et al.** *J Am. Chem. Soc.,* 1992, vol. 114, 9677
- **Murashige, T. ; Skoog, F.** *Physiologia Plantarum,* 1962, vol. 15, 473-497
- **Heller, R.** *Ann Sci Nat Bot Biol veg,* 1953, vol. 14, 1-223
- **Morel, G. ; R.H. Wetmore.** *Amer. J. Bot.,* 1951, vol. 38, 138-40

SEQUENCE LISTING

<110> Keygene NV

<120> Alternative nucleotides for targeted nucleotide exchange

<130> P27711pc01

<150> pct/nl2005/000884
<151> 2005-12-22

<160> 47

<170> PatentIn version 3.3

<210> 1
<211> 79
<212> DNA
<213> Nicotiana benthamiana

<400> 1
caacaatagg agtttcctga aaagcatcag tacctatcat cctacgttgc acttgacctg 60

ttatagcaac aatggggac 79

<210> 2
<211> 20
<212> DNA
<213> Artificial

<220>
<223> Primer for PCR amplification

<400> 2
ggtcaagtgc cacgtaggat 20

<210> 3
<211> 20
<212> DNA
<213> Artificial

<220>
<223> Primer for PCR amplification

<400> 3
gggtgcttca ctttctgctc 20

<210> 4
<211> 79
<212> DNA

<213> Artificial

<220>
<223> oligonucleotide for improved targeted nucleotide exchange

<400> 4
caacaatagg agtttcctga aaagcatcag tacctatcat cctacgttgc acttgacctg 60

ttatagcaac aatggggac 79

<210> 5
<211> 79
<212> DNA
<213> Artificial

<220>
<223> Modified oligonucleotide

<220>
<221> morpholinophosphoroamidate backbone
<222> (1)..(79)
<223> morpholinophosphoroamidate backbone

<220>
<221> modified_base
<222> (1)..(79)
<223> morpholinophosphoroamidate backbone

<400> 5
caacaatagg agtttcctga aaagcatcag tacctatcat cctacgttgc acttgacctg 60

ttatagcaac aatggggac 79

<210> 6
<211> 79
<212> DNA
<213> Artificial

<220>
<223> oligonucleotide for improved targeted nucleotide exchange

<220>
<221> modified_base
<222> (1)..(2)
<223> 2'-fluoroarabinonucleic acid

<220>
<221> modified_base
<222> (78)..(79)

<223>  2'-fluoroarabinonucleic acid

<400>  6
caacaatagg agtttcctga aaagcatcag tacctatcat cctacgttgc acttgacctg     60

ttatagcaac aatggggac                                                  79

<210>  7
<211>  79
<212>  DNA
<213>  Artificial

<220>
<223>  oligonucleotide for improved targeted nucleotide exchange

<220>
<221>  misc_feature
<222>  (1)..(1)
<223>  6-chloro-2-methoxyacridine

<220>
<221>  modified_base
<222>  (1)..(1)
<223>  6-chloro-2-methoxyacridine

<220>
<221>  modified_base
<222>  (79)..(79)
<223>  6-chloro-2-methoxyacridine

<400>  7
caacaatagg agtttcctga aaagcatcag tacctatcat cctacgttgc acttgacctg     60

ttatagcaac aatggggac                                                  79

<210>  8
<211>  79
<212>  DNA
<213>  Artificial

<220>
<223>  oligonucleotide for improved targeted nucleotide exchange

<220>
<221>  misc_feature
<222>  (46)..(46)
<223>  6-chloro-2-methoxyacridine

<220>

<221>  misc_feature
<222>  (49)..(49)
<223>  6-chloro-2-methoxyacridine

<400>  8
caacaatagg agtttcctga aaagcatcag tacctatcat cctacgttgc acttgacctg     60

ttatagcaac aatggggac                                                  79


<210>  9
<211>  79
<212>  DNA
<213>  Artificial

<220>
<223>  oligonucleotide for improved targeted nucleotide exchange


<220>
<221>  misc_feature
<222>  (46)..(47)
<223>  6-chloro-2-methoxyacridine

<220>
<221>  misc_feature
<222>  (49)..(50)
<223>  6-chloro-2-methoxyacridine

<400>  9
caacaatagg agtttcctga aaagcatcag tacctatcat cctacgttgc acttgacctg     60

ttatagcaac aatggggac                                                  79


<210>  10
<211>  79
<212>  DNA
<213>  Artificial

<220>
<223>  oligonucleotide for improved targeted nucleotide exchange


<220>
<221>  misc_feature
<222>  (45)..(45)
<223>  6-chloro-2-methoxyacridine

<220>
<221>  misc_feature
<222>  (50)..(50)
<223>  6-chloro-2-methoxyacridine

<400> 10
caacaatagg agtttcctga aaagcatcag tacctatcat cctacgttgc acttgacctg 60

ttatagcaac aatggggac 79

<210> 11
<211> 79
<212> DNA
<213> Artificial

<220>
<223> oligonucleotide for improved targeted nucleotide exchange

<220>
<221> misc_feature
<222> (1)..(1)
<223> 6-chloro-2-methoxyacridine

<220>
<221> misc_feature
<222> (46)..(46)
<223> 6-chloro-2-methoxyacridine

<220>
<221> misc_feature
<222> (49)..(49)
<223> 6-chloro-2-methoxyacridine

<220>
<221> misc_feature
<222> (79)..(79)
<223> 6-chloro-2-methoxyacridine

<400> 11
caacaatagg agtttcctga aaagcatcag tacctatcat cctacgttgc acttgacctg 60

ttatagcaac aatggggac 79

<210> 12
<211> 79
<212> DNA
<213> Artificial

<220>
<223> oligonucleotide for improved targeted nucleotide exchange

<220>
<221> modified_base

<222> (1)..(1)
<223> 6-chloro-2-methoxyacridine

<220>
<221> modified_base
<222> (45)..(46)
<223> 6-chloro-2-methoxyacridine

<220>
<221> modified_base
<222> (49)..(50)
<223> 6-chloro-2-methoxyacridine

<220>
<221> modified_base
<222> (79)..(79)
<223> 6-chloro-2-methoxyacridine

<400> 12
caacaatagg agtttcctga aaagcatcag tacctatcat cctacgttgc acttgacctg 60

ttatagcaac aatggggac 79

<210> 13
<211> 79
<212> DNA
<213> Artificial

<220>
<223> oligonucleotide for improved targeted nucleotide exchange

<220>
<221> modified_base
<222> (1)..(1)
<223> 6-chloro-2-methoxyacridine

<220>
<221> modified_base
<222> (45)..(45)
<223> 6-chloro-2-methoxyacridine

<220>
<221> modified_base
<222> (50)..(50)
<223> 6-chloro-2-methoxyacridine

<220>
<221> modified_base
<222> (79)..(79)
<223> 6-chloro-2-methoxyacridine

<400> 13
caacaatagg agtttcctga aaagcatcag tacctatcat cctacgttgc acttgacctg 60

ttatagcaac aatggggac 79

<210> 14
<211> 79
<212> DNA
<213> Artificial

<220>
<223> oligonucleotide for improved targeted nucleotide exchange

<220>
<221> modified_base
<222> (1)..(4)
<223> phosphorothioate linkage

<220>
<221> modified_base
<222> (76)..(79)
<223> phosphorothioate linkage

<400> 14
caacaatagg agtttcctga aaagcatcag tacctatcat cctacgttgc acttgacctg 60

ttatagcaac aatggggac 79

<210> 15
<211> 79
<212> DNA
<213> Artificial

<220>
<223> oligonucleotide for improved targeted nucleotide exchange

<220>
<221> modified_base
<222> (1)..(79)
<223> methylphosphonate linkage

<400> 15
caacaatagg agtttcctga aaagcatcag tacctatcat cctacgttgc acttgacctg 60

ttatagcaac aatggggac 79

<210> 16
<211> 79

<212> DNA
<213> Artificial

<220>
<223> oligonucleotide for improved targeted nucleotide exchange

<220>
<221> modified_base
<222> (2)..(2)
<223> ALPHA-LOCKED NUCLEIC ACID

<220>
<221> modified_base
<222> (4)..(4)
<223> ALPHA-LOCKED NUCLEIC ACID

<220>
<221> modified_base
<222> (6)..(6)
<223> ALPHA-LOCKED NUCLEIC ACID

<220>
<221> modified_base
<222> (8)..(8)
<223> ALPHA-LOCKED NUCLEIC ACID

<220>
<221> modified_base
<222> (10)..(10)
<223> ALPHA-LOCKED NUCLEIC ACID

<220>
<221> modified_base
<222> (12)..(12)
<223> ALPHA-LOCKED NUCLEIC ACID

<220>
<221> modified_base
<222> (14)..(14)
<223> ALPHA-LOCKED NUCLEIC ACID

<220>
<221> modified_base
<222> (16)..(16)
<223> ALPHA-LOCKED NUCLEIC ACID

<220>
<221> modified_base
<222> (18)..(18)
<223> ALPHA-LOCKED NUCLEIC ACID

<220>

<221> modified_base
<222> (20)..(20)
<223> ALPHA-LOCKED NUCLEIC ACID

<220>
<221> modified_base
<222> (20)..(20)
<223> ALPHA-LOCKED NUCLEIC ACID

<220>
<221> modified_base
<222> (22)..(22)
<223> ALPHA-LOCKED NUCLEIC ACID

<220>
<221> modified_base
<222> (24)..(24)
<223> ALPHA-LOCKED NUCLEIC ACID

<220>
<221> modified_base
<222> (26)..(26)
<223> ALPHA-LOCKED NUCLEIC ACID

<220>
<221> modified_base
<222> (28)..(28)
<223> ALPHA-LOCKED NUCLEIC ACID

<220>
<221> modified_base
<222> (30)..(30)
<223> ALPHA-LOCKED NUCLEIC ACID

<220>
<221> modified_base
<222> (32)..(32)
<223> ALPHA-LOCKED NUCLEIC ACID

<220>
<221> modified_base
<222> (34)..(34)
<223> ALPHA-LOCKED NUCLEIC ACID

<220>
<221> modified_base
<222> (36)..(36)
<223> ALPHA-LOCKED NUCLEIC ACID

<220>
<221> modified_base
<222> (38)..(38)
<223> ALPHA-LOCKED NUCLEIC ACID

```
<220>
<221>  modified_base
<222>  (40)..(40)
<223>  ALPHA-LOCKED NUCLEIC ACID

<220>
<221>  modified_base
<222>  (42)..(42)
<223>  ALPHA-LOCKED NUCLEIC ACID

<220>
<221>  modified_base
<222>  (44)..(44)
<223>  ALPHA-LOCKED NUCLEIC ACID

<220>
<221>  modified_base
<222>  (46)..(46)
<223>  ALPHA-LOCKED NUCLEIC ACID

<220>
<221>  modified_base
<222>  (48)..(48)
<223>  ALPHA-LOCKED NUCLEIC ACID

<220>
<221>  modified_base
<222>  (50)..(50)
<223>  ALPHA-LOCKED NUCLEIC ACID

<220>
<221>  modified_base
<222>  (52)..(52)
<223>  ALPHA-LOCKED NUCLEIC ACID

<220>
<221>  modified_base
<222>  (54)..(54)
<223>  ALPHA-LOCKED NUCLEIC ACID

<220>
<221>  modified_base
<222>  (56)..(56)
<223>  ALPHA-LOCKED NUCLEIC ACID

<220>
<221>  modified_base
<222>  (58)..(58)
<223>  ALPHA-LOCKED NUCLEIC ACID

<220>
<221>  modified_base
```

<222> (60)..(60)
<223> ALPHA-LOCKED NUCLEIC ACID

<220>
<221> modified_base
<222> (62)..(62)
<223> ALPHA-LOCKED NUCLEIC ACID

<220>
<221> modified_base
<222> (64)..(64)
<223> ALPHA-LOCKED NUCLEIC ACID

<220>
<221> modified_base
<222> (66)..(66)
<223> ALPHA-LOCKED NUCLEIC ACID

<220>
<221> modified_base
<222> (68)..(68)
<223> ALPHA-LOCKED NUCLEIC ACID

<220>
<221> modified_base
<222> (70)..(70)
<223> ALPHA-LOCKED NUCLEIC ACID

<220>
<221> modified_base
<222> (72)..(72)
<223> ALPHA-LOCKED NUCLEIC ACID

<220>
<221> modified_base
<222> (74)..(74)
<223> ALPHA-LOCKED NUCLEIC ACID

<220>
<221> modified_base
<222> (76)..(76)
<223> ALPHA-LOCKED NUCLEIC ACID

<220>
<221> modified_base
<222> (78)..(78)
<223> ALPHA-LOCKED NUCLEIC ACID

<400> 16
caacaatagg agtttcctga aaagcatcag tacctatcat cctacgttgc acttgacctg 60

ttatagcaac aatggggac 79

<210> 17
<211> 79
<212> DNA
<213> Artificial

<220>
<223> oligonucleotide for improved targeted nucleotide exchange

<220>
<221> modified_base
<222> (1)..(1)
<223> ALPHA-LOCKED NUCLEIC ACID

<220>
<221> modified_base
<222> (3)..(3)
<223> ALPHA-LOCKED NUCLEIC ACID

<220>
<221> modified_base
<222> (5)..(5)
<223> ALPHA-LOCKED NUCLEIC ACID

<220>
<221> modified_base
<222> (7)..(7)
<223> ALPHA-LOCKED NUCLEIC ACID

<220>
<221> modified_base
<222> (9)..(9)
<223> ALPHA-LOCKED NUCLEIC ACID

<220>
<221> modified_base
<222> (11)..(11)
<223> ALPHA-LOCKED NUCLEIC ACID

<220>
<221> modified_base
<222> (13)..(13)
<223> ALPHA-LOCKED NUCLEIC ACID

<220>
<221> modified_base
<222> (15)..(15)
<223> ALPHA-LOCKED NUCLEIC ACID

<220>
<221> modified_base
<222> (17)..(17)

<223> ALPHA-LOCKED NUCLEIC ACID

<220>
<221> modified_base
<222> (19)..(19)
<223> ALPHA-LOCKED NUCLEIC ACID

<220>
<221> modified_base
<222> (21)..(21)
<223> ALPHA-LOCKED NUCLEIC ACID

<220>
<221> modified_base
<222> (23)..(23)
<223> ALPHA-LOCKED NUCLEIC ACID

<220>
<221> modified_base
<222> (25)..(25)
<223> ALPHA-LOCKED NUCLEIC ACID

<220>
<221> modified_base
<222> (27)..(27)
<223> ALPHA-LOCKED NUCLEIC ACID

<220>
<221> modified_base
<222> (29)..(29)
<223> ALPHA-LOCKED NUCLEIC ACID

<220>
<221> modified_base
<222> (31)..(31)
<223> ALPHA-LOCKED NUCLEIC ACID

<220>
<221> modified_base
<222> (33)..(33)
<223> ALPHA-LOCKED NUCLEIC ACID

<220>
<221> modified_base
<222> (35)..(35)
<223> ALPHA-LOCKED NUCLEIC ACID

<220>
<221> modified_base
<222> (37)..(37)
<223> ALPHA-LOCKED NUCLEIC ACID

<220>

<221> modified_base
<222> (39)..(39)
<223> ALPHA-LOCKED NUCLEIC ACID

<220>
<221> modified_base
<222> (41)..(41)
<223> ALPHA-LOCKED NUCLEIC ACID

<220>
<221> modified_base
<222> (43)..(43)
<223> ALPHA-LOCKED NUCLEIC ACID

<220>
<221> modified_base
<222> (45)..(45)
<223> ALPHA-LOCKED NUCLEIC ACID

<220>
<221> modified_base
<222> (47)..(47)
<223> ALPHA-LOCKED NUCLEIC ACID

<220>
<221> modified_base
<222> (49)..(49)
<223> ALPHA-LOCKED NUCLEIC ACID

<220>
<221> modified_base
<222> (51)..(51)
<223> ALPHA-LOCKED NUCLEIC ACID

<220>
<221> modified_base
<222> (53)..(53)
<223> ALPHA-LOCKED NUCLEIC ACID

<220>
<221> modified_base
<222> (55)..(55)
<223> ALPHA-LOCKED NUCLEIC ACID

<220>
<221> modified_base
<222> (57)..(57)
<223> ALPHA-LOCKED NUCLEIC ACID

<220>
<221> modified_base
<222> (59)..(59)
<223> ALPHA-LOCKED NUCLEIC ACID

<220>
<221> modified_base
<222> (61)..(61)
<223> ALPHA-LOCKED NUCLEIC ACID

<220>
<221> modified_base
<222> (63)..(63)
<223> ALPHA-LOCKED NUCLEIC ACID

<220>
<221> modified_base
<222> (65)..(65)
<223> ALPHA-LOCKED NUCLEIC ACID

<220>
<221> modified_base
<222> (67)..(67)
<223> ALPHA-LOCKED NUCLEIC ACID

<220>
<221> modified_base
<222> (69)..(69)
<223> ALPHA-LOCKED NUCLEIC ACID

<220>
<221> modified_base
<222> (71)..(71)
<223> ALPHA-LOCKED NUCLEIC ACID

<220>
<221> modified_base
<222> (73)..(73)
<223> ALPHA-LOCKED NUCLEIC ACID

<220>
<221> modified_base
<222> (75)..(75)
<223> ALPHA-LOCKED NUCLEIC ACID

<220>
<221> modified_base
<222> (77)..(77)
<223> ALPHA-LOCKED NUCLEIC ACID

<220>
<221> modified_base
<222> (79)..(79)
<223> ALPHA-LOCKED NUCLEIC ACID

<400> 17
caacaatagg agtttcctga aaagcatcag tacctatcat cctacgttgc acttgacctg 60

ttatagcaac aatggggac 79

<210> 18
<211> 79
<212> DNA
<213> Artificial

<220>
<223> oligonucleotide for improved targeted nucleotide exchange

<220>
<221> modified_base
<222> (1)..(2)
<223> I

<220>
<221> modified_base
<222> (78)..(79)
<223> I

<400> 18
caacaatagg agtttcctga aaagcatcag tacctatcat cctacgttgc acttgacctg 60

ttatagcaac aatggggac 79

<210> 19
<211> 79
<212> DNA
<213> Artificial

<220>
<223> oligonucleotide for improved targeted nucleotide exchange

<220>
<221> modified_base
<222> (1)..(2)
<223> I

<220>
<221> modified_base
<222> (47)..(47)
<223> I

<220>
<221> modified_base
<222> (49)..(49)
<223> I

<220>
<221> modified_base
<222> (78)..(79)
<223> I

<400> 19
caacaatagg agtttcctga aaagcatcag tacctatcat cctacgttgc acttgacctg 60

ttatagcaac aatggggac 79

<210> 20
<211> 79
<212> DNA
<213> Artificial

<220>
<223> oligonucleotide for improved targeted nucleotide exchange

<220>
<221> modified_base
<222> (1)..(1)
<223> 2 fluoro RNA (C)

<220>
<221> modified_base
<222> (79)..(79)
<223> 2 fluoro RNA (C)

<400> 20
caacaatagg agtttcctga aaagcatcag tacctatcat cctacgttgc acttgacctg 60

ttatagcaac aatggggac 79

<210> 21
<211> 79
<212> DNA
<213> Artificial

<220>
<223> oligonucleotide for improved targeted nucleotide exchange

<220>
<221> modified_base
<222> (1)..(1)
<223> 2-fluoro RNA (C)

<220>
<221> modified_base
<222> (47)..(47)

<223> 2-fluoro RNA (C)

<220>
<221> modified_base
<222> (50)..(50)
<223> 2-fluoro RNA (C)

<220>
<221> modified_base
<222> (79)..(79)
<223> 2-fluoro RNA (C)

<400> 21
caacaatagg agtttcctga aaagcatcag tacctatcat cctacgttgc acttgacctg 60

ttatagcaac aatggggac 79

<210> 22
<211> 79
<212> DNA
<213> Artificial

<220>
<223> oligonucleotide for improved targeted nucleotide exchange

<220>
<221> modified_base
<222> (1)..(2)
<223> 2'-O-methoxyethyl RNA

<220>
<221> modified_base
<222> (78)..(79)
<223> 2'-O-methoxyethyl RNA

<400> 22
caacaatagg agtttcctga aaagcatcag tacctatcat cctacgttgc acttgacctg 60

ttatagcaac aatggggac 79

<210> 23
<211> 79
<212> DNA
<213> Artificial

<220>
<223> oligonucleotide for improved targeted nucleotide exchange

<220>

<221> modified_base
<222> (47)..(47)
<223> 2'-O-methoxyethyl RNA

<220>
<221> modified_base
<222> (49)..(49)
<223> 2'-O-methoxyethyl RNA

<400> 23
caacaatagg agtttcctga aaagcatcag tacctatcat cctacgttgc acttgacctg 60

ttatagcaac aatggggac 79

<210> 24
<211> 79
<212> DNA
<213> Artificial

<220>
<223> oligonucleotide for improved targeted nucleotide exchange

<220>
<221> modified_base
<222> (1)..(2)
<223> 2'-O-methoxyethyl RNA

<220>
<221> modified_base
<222> (47)..(47)
<223> 2'-O-methoxyethyl RNA

<220>
<221> modified_base
<222> (49)..(49)
<223> 2'-O-methoxyethyl RNA

<220>
<221> modified_base
<222> (78)..(79)
<223> 2'-O-methoxyethyl RNA

<400> 24
caacaatagg agtttcctga aaagcatcag tacctatcat cctacgttgc acttgacctg 60

ttatagcaac aatggggac 79

<210> 25
<211> 79
<212> DNA

<213>  Artificial

<220>
<223>  oligonucleotide for improved targeted nucleotide exchange

<220>
<221>  modified_base
<222>  (1)..(79)
<223>  N3-P5-phosphoroamidate linkages

<400>  25
caacaatagg agtttcctga aaagcatcag tacctatcat cctacgttgc acttgacctg     60

ttatagcaac aatggggac                                                  79

<210>  26
<211>  79
<212>  DNA
<213>  Artificial

<220>
<223>  oligonucleotide for improved targeted nucleotide exchange

<220>
<221>  modified_base
<222>  (44)..(44)
<223>  2-AMINOADENOSINE

<220>
<221>  modified_base
<222>  (51)..(51)
<223>  2-AMINOADENOSINE

<400>  26
caacaatagg agtttcctga aaagcatcag tacctatcat cctacgttgc acttgacctg     60

ttatagcaac aatggggac                                                  79

<210>  27
<211>  79
<212>  DNA
<213>  Artificial

<220>
<223>  oligonucleotide for improved targeted nucleotide exchange

<220>
<221>  modified_base

<222> (1)..(79)
<223> super A from epoch biosciences

<400> 27
caacaatagg agtttcctga aaagcatcag tacctatcat cctacgttgc acttgacctg 60

ttatagcaac aatggggac 79

<210> 28
<211> 79
<212> DNA
<213> Artificial

<220>
<223> oligonucleotide for improved targeted nucleotide exchange

<220>
<221> modified_base
<222> (1)..(79)
<223> super T from epoch biosciences

<400> 28
caacaatagg agtttcctga aaagcatcag tacctatcat cctacgttgc acttgacctg 60

ttatagcaac aatggggac 79

<210> 29
<211> 79
<212> DNA
<213> Artificial

<220>
<223> oligonucleotide for improved targeted nucleotide exchange

<220>
<221> modified_base
<222> (42)..(42)
<223> 5-methylisodeoxycytidine

<220>
<221> modified_base
<222> (45)..(45)
<223> 5-methylisodeoxycytidine

<220>
<221> modified_base
<222> (50)..(50)
<223> 5-methylisodeoxycytidine

<220>
<221> modified_base
<222> (52)..(52)
<223> 5-methylisodeoxycytidine

<400> 29
caacaatagg agtttcctga aaagcatcag tacctatcat cctacgttgc acttgacctg 60

ttatagcaac aatggggac 79

<210> 30
<211> 79
<212> DNA
<213> Artificial

<220>
<223> oligonucleotide for improved targeted nucleotide exchange

<220>
<221> modified_base
<222> (47)..(47)
<223> 5-propynyl-2-deoxynucleoside

<220>
<221> modified_base
<222> (49)..(49)
<223> 5-propynyl-2-deoxynucleoside

<400> 30
caacaatagg agtttcctga aaagcatcag tacctatcat cctacgttgc acttgacctg 60

ttatagcaac aatggggac 79

<210> 31
<211> 79
<212> DNA
<213> Artificial

<220>
<223> oligonucleotide for improved targeted nucleotide exchange

<220>
<221> modified_base
<222> (46)..(46)
<223> 5-propynyl-2-deoxynucleoside

<220>
<221> modified_base
<222> (50)..(50)

<223>  5-propynyl-2-deoxynucleoside

<400>  31
caacaatagg agtttcctga aaagcatcag tacctatcat cctacgttgc acttgacctg    60

ttatagcaac aatggggac    79

<210>  32
<211>  79
<212>  DNA
<213>  Artificial

<220>
<223>  oligonucleotide for improved targeted nucleotide exchange

<220>
<221>  modified_base
<222>  (46)..(47)
<223>  5-propynyl-2-deoxynucleoside

<220>
<221>  modified_base
<222>  (49)..(50)
<223>  5-propynyl-2-deoxynucleoside

<400>  32
caacaatagg agtttcctga aaagcatcag tacctatcat cctacgttgc acttgacctg    60

ttatagcaac aatggggac    79

<210>  33
<211>  79
<212>  DNA
<213>  Artificial

<220>
<223>  oligonucleotide for improved targeted nucleotide exchange

<220>
<221>  modified_base
<222>  (47)..(47)
<223>  N4-ethyl-2'-deoxycytidine

<220>
<221>  modified_base
<222>  (49)..(49)
<223>  N4-ethyl-2'deoxycytidine

<400>  33

caacaatagg agtttcctga aaagcatcag tacctatcat cctacgttgc acttgacctg 60

ttatagcaac aatggggac 79

<210> 34
<211> 79
<212> DNA
<213> Artificial

<220>
<223> oligonucleotide for improved targeted nucleotide exchange

<220>
<221> modified_base
<222> (46)..(46)
<223> N4-ethyl-2'deoxycytidine

<220>
<221> modified_base
<222> (50)..(50)
<223> N4-ethyl-2'deoxycytidine

<400> 34
caacaatagg agtttcctga aaagcatcag tacctatcat cctacgttgc acttgacctg 60

ttatagcaac aatggggac 79

<210> 35
<211> 79
<212> DNA
<213> Artificial

<220>
<223> oligonucleotide for improved targeted nucleotide exchange

<220>
<221> modified_base
<222> (46)..(47)
<223> N4-ethyl-2'deoxycytidine

<220>
<221> modified_base
<222> (49)..(50)
<223> N4-ethyl-2'deoxycytidine

<400> 35
caacaatagg agtttcctga aaagcatcag tacctatcat cctacgttgc acttgacctg 60

ttatagcaac aatggggac 79

<210> 36
<211> 24
<212> DNA
<213> Artificial

<220>
<223> oligonucleotide for improved targeted nucleotide exchange

<400> 36
tgtgcccagt cgtagccgaa tagc 24

<210> 37
<211> 24
<212> DNA
<213> Artificial

<220>
<223> Modified oligonucleotide

<220>
<221> modified_base
<222> (1)..(1)
<223> LOCKED NUCLEIC ACID

<220>
<221> modified_base
<222> (24)..(24)
<223> LOCKED NUCLEIC ACID

<400> 37
tgtgcccagt cgtagccgaa tagc 24

<210> 38
<211> 24
<212> DNA
<213> Artificial

<220>
<223> oligonucleotide for improved targeted nucleotide exchange

<220>
<221> modified_base
<222> (1)..(2)
<223> LOCKED NUCLEIC ACID

<220>
<221> modified_base

<222> (23)..(24)
<223> LOCKED NUCLEIC ACID

<400> 38
tgtgcccagt cgtagccgaa tagc 24

<210> 39
<211> 24
<212> DNA
<213> artificial

<220>
<223> oligonucleotide for improved targeted nucleotide exchange

<220>
<221> modified_base
<222> (1)..(3)
<223> LOCKED NUCLEIC ACID

<220>
<221> modified_base
<222> (22)..(24)
<223> LOCKED NUCLEIC ACID

<400> 39
tgtgcccagt cgtagccgaa tagc 24

<210> 40
<211> 24
<212> DNA
<213> artificial

<220>
<223> oligonucleotide for improved targeted nucleotide exchange

<220>
<221> modified_base
<222> (1)..(4)
<223> LOCKED NUCLEIC ACID

<220>
<221> modified_base
<222> (21)..(24)
<223> LOCKED NUCLEIC ACID

<400> 40
tgtgcccagt cgtagccgaa tagc 24

<210> 41
<211> 24
<212> DNA
<213> artificial

<220>
<223> oligonucleotide for improved targeted nucleotide exchange

<220>
<221> modified_base
<222> (10)..(10)
<223> LOCKED NUCLEIC ACID

<220>
<221> modified_base
<222> (14)..(14)
<223> LOCKED NUCLEIC ACID

<400> 41
tgtgcccagt cgtagccgaa tagc 24

<210> 42
<211> 24
<212> DNA
<213> artificial

<220>
<223> oligonucleotide for improved targeted nucleotide exchange

<220>
<221> modified_base
<222> (9)..(9)
<223> Locked nucleic acid

<220>
<221> modified_base
<222> (15)..(15)
<223> Locked nucleic acid

<400> 42
tgtgcccagt cgtagccgaa tagc 24

<210> 43
<211> 24
<212> DNA
<213> artificial

<220>
<223> oligonucleotide for improved targeted nucleotide exchange

<220>
<221> modified_base
<222> (8)..(8)
<223> lOCKED NUCLEIC ACID

<220>
<221> modified_base
<222> (16)..(16)
<223> lOCKED NUCLEIC ACID

<400> 43
tgtgcccagt cgtagccgaa tagc 24

<210> 44
<211> 24
<212> DNA
<213> artificial

<220>
<223> oligonucleotide for improved targeted nucleotide exchange

<220>
<221> modified_base
<222> (8)..(8)
<223> LOCKED NUCLEIC ACID

<220>
<221> modified_base
<222> (10)..(10)
<223> LOCKED NUCLEIC ACID

<220>
<221> modified_base
<222> (14)..(14)
<223> LOCKED NUCLEIC ACID

<220>
<221> modified_base
<222> (16)..(16)
<223> LOCKED NUCLEIC ACID

<400> 44
tgtgcccagt cgtagccgaa tagc 24

<210> 45
<211> 24
<212> DNA
<213> artificial

<220>
<223> oligonucleotide for improved targeted nucleotide exchange

<220>
<221> modified_base
<222> (11)..(11)
<223> LOCKED NUCLEIC ACID

<220>
<221> modified_base
<222> (13)..(13)
<223> LOCKED NUCLEIC ACID

<400> 45
tgtgcccagt cgtagccgaa tagc 24

<210> 46
<211> 24
<212> DNA
<213> artificial

<220>
<223> oligonucleotide for improved targeted nucleotide exchange

<220>
<221> modified_base
<222> (11)..(11)
<223> N4-ethyl-deoxycytidine

<220>
<221> modified_base
<222> (16)..(16)
<223> N4-ethyl-deoxycytidine

<400> 46
tgtgcccagt cgtagccgaa tagc 24

<210> 47
<211> 24
<212> DNA
<213> artificial

<220>
<223> oligonucleotide for improved targeted nucleotide exchange

<220>
<221> modified_base

<222> (11)..(11)
<223> 5-propynyl-2-deoxycytidine

<220>
<221> modified_base
<222> (13)..(13)
<223> 5-propynyl-2-deoxyuracil

<400> 47
tgtgcccagt cgtagccgaa tagc 24

SEQUENCE LISTING

<110> Keygene NV

<120> Alternative nucleotides for targeted nucleotide exchange

<130> P27711EP03

<150> pct/nl2005/000884
<151> 2005-12-22

<160> 47

<170> PatentIn version 3.3

<210> 1
<211> 79
<212> DNA
<213> Nicotiana benthamiana

<400> 1
caacaatagg agtttcctga aaagcatcag tacctatcat cctacgttgc acttgacctg 60

ttatagcaac aatggggac 79

<210> 2
<211> 20
<212> DNA
<213> Artificial

<220>
<223> Primer for PCR amplification

<400> 2
ggtcaagtgc cacgtaggat 20

<210> 3
<211> 20
<212> DNA
<213> Artificial

<220>
<223> Primer for PCR amplification

<400> 3
gggtgcttca ctttctgctc 20

<210> 4
<211> 79
<212> DNA
<213> Artificial

<220>
<223> oligonucleotide for improved targeted nucleotide exchange

<400> 4
caacaatagg agtttcctga aaagcatcag tacctatcat cctacgttgc acttgacctg 60

ttatagcaac aatggggac 79

<210> 5
<211> 79
<212> DNA
<213> Artificial

<220>
<223> Modified oligonucleotide

<220>
<221> morpholinophosphoroamidate backbone
<222> (1)..(79)
<223> morpholinophosphoroamidate backbone

<220>
<221> modified_base
<222> (1)..(79)
<223> morpholinophosphoroamidate backbone

<400> 5
caacaatagg agtttcctga aaagcatcag tacctatcat cctacgttgc acttgacctg 60

ttatagcaac aatggggac 79

<210> 6
<211> 79
<212> DNA
<213> Artificial

<220>
<223> oligonucleotide for improved targeted nucleotide exchange

<220>
<221> modified_base
<222> (1)..(2)
<223> 2'-fluoroarabinonucleic acid

<220>
<221> modified_base
<222> (78)..(79)
<223> 2'-fluoroarabinonucleic acid

<400> 6
caacaatagg agtttcctga aaagcatcag tacctatcat cctacgttgc acttgacctg 60

ttatagcaac aatggggac 79

<210> 7
<211> 79
<212> DNA
<213> Artificial

<220>
<223> oligonucleotide for improved targeted nucleotide exchange

<220>
<221> misc_feature
<222> (1)..(1)
<223> 6-chloro-2-methoxyacridine

<220>
<221> modified_base
<222> (1)..(1)
<223> 6-chloro-2-methoxyacridine

<220>
<221> modified_base
<222> (79)..(79)
<223> 6-chloro-2-methoxyacridine

<400> 7
caacaatagg agtttcctga aaagcatcag tacctatcat cctacgttgc acttgacctg 60

ttatagcaac aatggggac 79

<210> 8
<211> 79
<212> DNA
<213> Artificial

<220>
<223> oligonucleotide for improved targeted nucleotide exchange

<220>
<221> misc_feature
<222> (46)..(46)
<223> 6-chloro-2-methoxyacridine

<220>
<221> misc_feature
<222> (49)..(49)
<223> 6-chloro-2-methoxyacridine

<400> 8
caacaatagg agtttcctga aaagcatcag tacctatcat cctacgttgc acttgacctg 60

ttatagcaac aatggggac 79

<210> 9
<211> 79
<212> DNA
<213> Artificial

<220>
<223> oligonucleotide for improved targeted nucleotide exchange

<220>
<221> misc_feature
<222> (46)..(47)
<223> 6-chloro-2-methoxyacridine

<220>
<221> misc_feature
<222> (49)..(50)
<223> 6-chloro-2-methoxyacridine

<400> 9
caacaatagg agtttcctga aaagcatcag tacctatcat cctacgttgc acttgacctg 60

ttatagcaac aatggggac 79

<210> 10
<211> 79
<212> DNA
<213> Artificial

<220>
<223> oligonucleotide for improved targeted nucleotide exchange

<220>
<221> misc_feature
<222> (45)..(45)

<220>
<221> misc_feature
<222> (50)..(50)
<223> 6-chloro-2-methoxyacridine

<400> 10
caacaatagg agtttcctga aaagcatcag tacctatcat cctacgttgc acttgacctg 60

ttatagcaac aatggggac 79

<210> 11
<211> 79
<212> DNA
<213> Artificial

<220>
<223> oligonucleotide for improved targeted nucleotide exchange

<220>
<221> misc_feature
<222> (1)..(1)
<223> 6-chloro-2-methoxyacridine

<220>
<221> misc_feature
<222> (46)..(46)
<223> 6-chloro-2-methoxyacridine

<220>
<221> misc_feature
<222> (49)..(49)
<223> 6-chloro-2-methoxyacridine

<220>
<221> misc_feature
<222> (79)..(79)
<223> 6-chloro-2-methoxyacridine

<400> 11
caacaatagg agtttcctga aaagcatcag tacctatcat cctacgttgc acttgacctg 60

ttatagcaac aatggggac 79

<210> 12
<211> 79
<212> DNA
<213> Artificial

<220>
<223> oligonucleotide for improved targeted nucleotide exchange

<220>
<221> modified_base
<222> (1)..(1)
<223> 6-chloro-2-methoxyacridine

<220>
<221> modified_base
<222> (45)..(46)
<223> 6-chloro-2-methoxyacridine

<220>
<221> modified_base
<222> (49)..(50)

<223> 6-chloro-2-methoxyacridine

<220>
<221> modified_base
<222> (79)..(79)
<223> 6-chloro-2-methoxyacridine

<400> 12
caacaatagg agtttcctga aaagcatcag tacctatcat cctacgttgc acttgacctg 60

ttatagcaac aatggggac 79

<210> 13
<211> 79
<212> DNA
<213> Artificial

<220>
<223> oligonucleotide for improved targeted nucleotide exchange

<220>
<221> modified_base
<222> (1)..(1)
<223> 6-chloro-2-methoxyacridine

<220>
<221> modified_base
<222> (45)..(45)
<223> 6-chloro-2-methoxyacridine

<220>
<221> modified_base
<222> (50)..(50)
<223> 6-chloro-2-methoxyacridine

<220>
<221> modified_base
<222> (79)..(79)
<223> 6-chloro-2-methoxyacridine

<400> 13
caacaatagg agtttcctga aaagcatcag tacctatcat cctacgttgc acttgacctg 60

ttatagcaac aatggggac 79

<210> 14
<211> 79
<212> DNA
<213> Artificial

<220>
<223> oligonucleotide for improved targeted nucleotide exchange

<220>
<221> modified_base
<222> (1)..(4)
<223> phosphorothioate linkage

<220>
<221> modified_base
<222> (76)..(79)
<223> phosphorothioate linkage

<400> 14
caacaatagg agtttcctga aaagcatcag tacctatcat cctacgttgc acttgacctg 60

ttatagcaac aatggggac 79

<210> 15
<211> 79
<212> DNA
<213> Artificial

<220>
<223> oligonucleotide for improved targeted nucleotide exchange

<220>
<221> modified_base
<222> (1)..(79)
<223> methylphosphonate linkage

<400> 15
caacaatagg agtttcctga aaagcatcag tacctatcat cctacgttgc acttgacctg 60

ttatagcaac aatggggac 79

<210> 16
<211> 79
<212> DNA
<213> Artificial

<220>
<223> oligonucleotide for improved targeted nucleotide exchange

<220>
<221> modified_base
<222> (2)..(2)
<223> ALPHA-LOCKED NUCLEIC ACID

<220>
<221> modified_base
<222> (4)..(4)
<223> ALPHA-LOCKED NUCLEIC ACID

<220>
<221> modified_base
<222> (6)..(6)
<223> ALPHA-LOCKED NUCLEIC ACID

<220>
<221> modified_base
<222> (8)..(8)
<223> ALPHA-LOCKED NUCLEIC ACID

<220>
<221> modified_base
<222> (10)..(10)
<223> ALPHA-LOCKED NUCLEIC ACID

<220>
<221> modified_base
<222> (12)..(12)
<223> ALPHA-LOCKED NUCLEIC ACID

<220>
<221> modified_base
<222> (14)..(14)
<223> ALPHA-LOCKED NUCLEIC ACID

<220>

<221> modified_base
<222> (16)..(16)
<223> ALPHA-LOCKED NUCLEIC ACID

<220>
<221> modified_base
<222> (18)..(18)
<223> ALPHA-LOCKED NUCLEIC ACID

<220>
<221> modified_base
<222> (20)..(20)
<223> ALPHA-LOCKED NUCLEIC ACID

<220>
<221> modified_base
<222> (20)..(20)
<223> ALPHA-LOCKED NUCLEIC ACID

<220>
<221> modified_base
<222> (22)..(22)
<223> ALPHA-LOCKED NUCLEIC ACID

<220>
<221> modified_base
<222> (24)..(24)
<223> ALPHA-LOCKED NUCLEIC ACID

<220>
<221> modified_base
<222> (26)..(26)
<223> ALPHA-LOCKED NUCLEIC ACID

<220>
<221> modified_base
<222> (28)..(28)
<223> ALPHA-LOCKED NUCLEIC ACID

<220>
<221> modified_base
<222> (30)..(30)
<223> ALPHA-LOCKED NUCLEIC ACID

<220>
<221> modified_base
<222> (32)..(32)
<223> ALPHA-LOCKED NUCLEIC ACID

<220>
<221> modified_base
<222> (34)..(34)
<223> ALPHA-LOCKED NUCLEIC ACID

<220>
<221> modified_base
<222> (36)..(36)
<223> ALPHA-LOCKED NUCLEIC ACID

<220>
<221> modified_base
<222> (38)..(38)
<223> ALPHA-LOCKED NUCLEIC ACID

<220>
<221> modified_base
<222> (40)..(40)
<223> ALPHA-LOCKED NUCLEIC ACID

<220>
<221> modified_base
<222> (42)..(42)
<223> ALPHA-LOCKED NUCLEIC ACID

<220>
<221> modified_base
<222> (44)..(44)
<223> ALPHA-LOCKED NUCLEIC ACID

<220>
<221> modified_base
<222> (46)..(46)
<223> ALPHA-LOCKED NUCLEIC ACID

<220>
<221> modified_base
<222> (48)..(48)
<223> ALPHA-LOCKED NUCLEIC ACID

<220>
<221> modified_base
<222> (50)..(50)
<223> ALPHA-LOCKED NUCLEIC ACID

<220>
<221> modified_base
<222> (52)..(52)
<223> ALPHA-LOCKED NUCLEIC ACID

<220>
<221> modified_base
<222> (54)..(54)
<223> ALPHA-LOCKED NUCLEIC ACID

<220>
<221> modified_base
<222> (56)..(56)
<223> ALPHA-LOCKED NUCLEIC ACID

<220>
<221> modified_base
<222> (58)..(58)
<223> ALPHA-LOCKED NUCLEIC ACID

<220>
<221> modified_base
<222> (60)..(60)
<223> ALPHA-LOCKED NUCLEIC ACID

<220>
<221> modified_base
<222> (62)..(62)
<223> ALPHA-LOCKED NUCLEIC ACID

<220>
<221> modified_base
<222> (64)..(64)
<223> ALPHA-LOCKED NUCLEIC ACID

<220>
<221> modified_base
<222> (66)..(66)
<223> ALPHA-LOCKED NUCLEIC ACID

<220>
<221> modified_base
<222> (68)..(68)
<223> ALPHA-LOCKED NUCLEIC ACID

<220>
<221> modified_base
<222> (70)..(70)
<223> ALPHA-LOCKED NUCLEIC ACID

<220>
<221> modified_base
<222> (72)..(72)
<223> ALPHA-LOCKED NUCLEIC ACID

<220>
<221> modified_base
<222> (74)..(74)
<223> ALPHA-LOCKED NUCLEIC ACID

<220>
<221> modified_base
<222> (76)..(76)
<223> ALPHA-LOCKED NUCLEIC ACID

<220>
<221> modified_base
<222> (78)..(78)
<223> ALPHA-LOCKED NUCLEIC ACID

<400> 16
caacaatagg agtttcctga aaagcatcag tacctatcat cctacgttgc acttgacctg 60

ttatagcaac aatggggac 79

<210> 17
<211> 79
<212> DNA
<213> Artificial

<220>
<223> oligonucleotide for improved targeted nucleotide exchange

<220>
<221> modified_base
<222> (1)..(1)
<223> ALPHA-LOCKED NUCLEIC ACID

<220>
<221> modified_base
<222> (3)..(3)
<223> ALPHA-LOCKED NUCLEIC ACID

<220>
<221> modified_base
<222> (5)..(5)
<223> ALPHA-LOCKED NUCLEIC ACID

<220>
<221> modified_base
<222> (7)..(7)
<223> ALPHA-LOCKED NUCLEIC ACID

<220>
<221> modified_base
<222> (9)..(9)
<223> ALPHA-LOCKED NUCLEIC ACID

<220>
<221> modified_base
<222> (11)..(11)

<223> ALPHA-LOCKED NUCLEIC ACID

<220>
<221> modified_base
<222> (13)..(13)
<223> ALPHA-LOCKED NUCLEIC ACID

<220>
<221> modified_base
<222> (15)..(15)
<223> ALPHA-LOCKED NUCLEIC ACID

<220>
<221> modified_base
<222> (17)..(17)
<223> ALPHA-LOCKED NUCLEIC ACID

<220>
<221> modified_base
<222> (19)..(19)
<223> ALPHA-LOCKED NUCLEIC ACID

<220>
<221> modified_base
<222> (21)..(21)
<223> ALPHA-LOCKED NUCLEIC ACID

<220>
<221> modified_base
<222> (23)..(23)
<223> ALPHA-LOCKED NUCLEIC ACID

<220>
<221> modified_base
<222> (25)..(25)
<223> ALPHA-LOCKED NUCLEIC ACID

<220>
<221> modified_base
<222> (27)..(27)
<223> ALPHA-LOCKED NUCLEIC ACID

<220>
<221> modified_base
<222> (29)..(29)
<223> ALPHA-LOCKED NUCLEIC ACID

<220>
<221> modified_base
<222> (31)..(31)
<223> ALPHA-LOCKED NUCLEIC ACID

<220>
<221> modified_base
<222> (33)..(33)
<223> ALPHA-LOCKED NUCLEIC ACID

<220>
<221> modified_base
<222> (35)..(35)
<223> ALPHA-LOCKED NUCLEIC ACID

<220>
<221> modified_base
<222> (37)..(37)
<223> ALPHA-LOCKED NUCLEIC ACID

<220>
<221> modified_base

<222> (39)..(39)
<223> ALPHA-LOCKED NUCLEIC ACID

<220>
<221> modified_base
<222> (41)..(41)
<223> ALPHA-LOCKED NUCLEIC ACID

<220>
<221> modified_base
<222> (43)..(43)
<223> ALPHA-LOCKED NUCLEIC ACID

<220>
<221> modified_base
<222> (45)..(45)
<223> ALPHA-LOCKED NUCLEIC ACID

<220>
<221> modified_base
<222> (47)..(47)
<223> ALPHA-LOCKED NUCLEIC ACID

<220>
<221> modified_base
<222> (49)..(49)
<223> ALPHA-LOCKED NUCLEIC ACID

<220>
<221> modified_base
<222> (51)..(51)
<223> ALPHA-LOCKED NUCLEIC ACID

<220>
<221> modified_base
<222> (53)..(53)
<223> ALPHA-LOCKED NUCLEIC ACID

<220>
<221> modified_base
<222> (55)..(55)
<223> ALPHA-LOCKED NUCLEIC ACID

<220>
<221> modified_base
<222> (57)..(57)
<223> ALPHA-LOCKED NUCLEIC ACID

<220>
<221> modified_base
<222> (59)..(59)
<223> ALPHA-LOCKED NUCLEIC ACID

<220>
<221> modified_base
<222> (61)..(61)
<223> ALPHA-LOCKED NUCLEIC ACID

<220>
<221> modified_base
<222> (63)..(63)
<223> ALPHA-LOCKED NUCLEIC ACID

<220>
<221> modified_base
<222> (65)..(65)
<223> ALPHA-LOCKED NUCLEIC ACID

<220>

<221> modified_base
<222> (67)..(67)
<223> ALPHA-LOCKED NUCLEIC ACID

<220>
<221> modified_base
<222> (69)..(69)
<223> ALPHA-LOCKED NUCLEIC ACID

<220>
<221> modified_base
<222> (71)..(71)
<223> ALPHA-LOCKED NUCLEIC ACID

<220>
<221> modified_base
<222> (73)..(73)
<223> ALPHA-LOCKED NUCLEIC ACID

<220>
<221> modified_base
<222> (75)..(75)
<223> ALPHA-LOCKED NUCLEIC ACID

<220>
<221> modified_base
<222> (77)..(77)
<223> ALPHA-LOCKED NUCLEIC ACID

<220>
<221> modified_base
<222> (79)..(79)
<223> ALPHA-LOCKED NUCLEIC ACID

<400> 17
caacaatagg agtttcctga aaagcatcag tacctatcat cctacgttgc acttgacctg 60

ttatagcaac aatggggac 79

<210> 18
<211> 79
<212> DNA
<213> Artificial

<220>
<223> oligonucleotide for improved targeted nucleotide exchange

<220>
<221> modified_base
<222> (1)..(2)
<223> I

<220>
<221> modified_base
<222> (78)..(79)
<223> I

<400> 18
caacaatagg agtttcctga aaagcatcag tacctatcat cctacgttgc acttgacctg 60

ttatagcaac aatggggac 79

<210> 19
<211> 79
<212> DNA
<213> Artificial

<220>
<223> oligonucleotide for improved targeted nucleotide exchange

<220>
<221> modified_base
<222> (1)..(2)
<223> I

<220>
<221> modified_base
<222> (47)..(47)
<223> I

<220>
<221> modified_base
<222> (49)..(49)
<223> I

<220>
<221> modified_base
<222> (78)..(79)
<223> I

<400> 19
caacaatagg agtttcctga aaagcatcag tacctatcat cctacgttgc acttgacctg 60

ttatagcaac aatggggac 79

<210> 20
<211> 79
<212> DNA
<213> Artificial

<220>
<223> oligonucleotide for improved targeted nucleotide exchange

<220>
<221> modified_base
<222> (1)..(1)
<223> 2 fluoro RNA (C)

<220>
<221> modified_base
<222> (79)..(79)
<223> 2 fluoro RNA (C)

<400> 20
caacaatagg agtttcctga aaagcatcag tacctatcat cctacgttgc acttgacctg 60

ttatagcaac aatggggac 79

<210> 21
<211> 79
<212> DNA
<213> Artificial

<220>
<223> oligonucleotide for improved targeted nucleotide exchange

<220>
<221> modified_base
<222> (1)..(1)
<223> 2-fluoro RNA (C)

<220>
<221> modified_base
<222> (47)..(47)
<223> 2-fluoro RNA (C)

<220>
<221> modified_base
<222> (50)..(50)
<223> 2-fluoro RNA (C)

<220>
<221> modified_base
<222> (79)..(79)
<223> 2-fluoro RNA (C)

<400> 21
caacaatagg agtttcctga aaagcatcag tacctatcat cctacgttgc acttgacctg 60

ttatagcaac aatggggac 79

<210> 22
<211> 79
<212> DNA
<213> Artificial

<220>
<223> oligonucleotide for improved targeted nucleotide exchange

<220>
<221> modified_base
<222> (1)..(2)
<223> 2'-O-methoxyethyl RNA

<220>
<221> modified_base
<222> (78)..(79)
<223> 2'-O-methoxyethyl RNA

<400> 22
caacaatagg agtttcctga aaagcatcag tacctatcat cctacgttgc acttgacctg 60

ttatagcaac aatggggac 79

<210> 23
<211> 79
<212> DNA
<213> Artificial

<220>
<223> oligonucleotide for improved targeted nucleotide exchange

<220>
<221> modified_base
<222> (47)..(47)
<223> 2'-O-methoxyethyl RNA

<220>
<221> modified_base
<222> (49)..(49)
<223> 2'-O-methoxyethyl RNA

<400> 23
caacaatagg agtttcctga aaagcatcag tacctatcat cctacgttgc acttgacctg 60

ttatagcaac aatggggac 79

<210> 24
<211> 79
<212> DNA
<213> Artificial

<220>
<223> oligonucleotide for improved targeted nucleotide exchange

<220>
<221> modified_base
<222> (1)..(2)
<223> 2'-O-methoxyethyl RNA

<220>
<221> modified_base
<222> (47)..(47)
<223> 2'-O-methoxyethyl RNA

<220>
<221> modified_base
<222> (49)..(49)
<223> 2'-O-methoxyethyl RNA

<220>
<221> modified_base
<222> (78)..(79)
<223> 2'-O-methoxyethyl RNA

<400> 24
caacaatagg agtttcctga aaagcatcag tacctatcat cctacgttgc acttgacctg 60

ttatagcaac aatggggac 79

<210> 25
<211> 79
<212> DNA
<213> Artificial

<220>
<223> oligonucleotide for improved targeted nucleotide exchange

<220>
<221> modified_base
<222> (1)..(79)
<223> N3-P5-phosphoroamidate linkages

<400> 25
caacaatagg agtttcctga aaagcatcag tacctatcat cctacgttgc acttgacctg 60

ttatagcaac aatggggac 79

<210> 26
<211> 79
<212> DNA
<213> Artificial

<220>
<223> oligonucleotide for improved targeted nucleotide exchange

<220>
<221> modified_base

<222> (44)..(44)
<223> 2-AMINOADENOSINE

<220>
<221> modified_base
<222> (51)..(51)
<223> 2-AMINOADENOSINE

<400> 26
caacaatagg agtttcctga aaagcatcag tacctatcat cctacgttgc acttgacctg 60

ttatagcaac aatggggac 79

<210> 27
<211> 79
<212> DNA
<213> Artificial

<220>
<223> oligonucleotide for improved targeted nucleotide exchange

<220>
<221> modified_base
<222> (1)..(79)
<223> super A from epoch biosciences

<400> 27
caacaatagg agtttcctga aaagcatcag tacctatcat cctacgttgc acttgacctg 60

ttatagcaac aatggggac 79

<210> 28
<211> 79
<212> DNA
<213> Artificial

<220>
<223> oligonucleotide for improved targeted nucleotide exchange

<220>
<221> modified_base
<222> (1)..(79)
<223> super T from epoch biosciences

<400> 28
caacaatagg agtttcctga aaagcatcag tacctatcat cctacgttgc acttgacctg 60

ttatagcaac aatggggac 79

<210> 29
<211> 79
<212> DNA
<213> Artificial

<220>
<223> oligonucleotide for improved targeted nucleotide exchange

<220>
<221> modified_base
<222> (42)..(42)
<223> 5-methylisodeoxycytidine

<220>

<221> modified_base
<222> (45)..(45)
<223> 5-methylisodeoxycytidine

<220>
<221> modified_base
<222> (50)..(50)
<223> 5-methylisodeoxycytidine

<220>
<221> modified_base
<222> (52)..(52)
<223> 5-methylisodeoxycytidine

<400> 29
caacaatagg agtttcctga aaagcatcag tacctatcat cctacgttgc acttgacctg 60

ttatagcaac aatggggac 79

<210> 30
<211> 79
<212> DNA
<213> Artificial

<220>
<223> oligonucleotide for improved targeted nucleotide exchange

<220>
<221> modified_base
<222> (47)..(47)
<223> 5-propynyl-2-deoxynucleoside

<220>
<221> modified_base
<222> (49)..(49)
<223> 5-propynyl-2-deoxynucleoside

<400> 30
caacaatagg agtttcctga aaagcatcag tacctatcat cctacgttgc acttgacctg 60

ttatagcaac aatggggac 79

<210> 31
<211> 79
<212> DNA
<213> Artificial

<220>
<223> oligonucleotide for improved targeted nucleotide exchange

<220>
<221> modified_base
<222> (46)..(46)
<223> 5-propynyl-2-deoxynucleoside

<220>
<221> modified_base
<222> (50)..(50)
<223> 5-propynyl-2-deoxynucleoside

<400> 31
caacaatagg agtttcctga aaagcatcag tacctatcat cctacgttgc acttgacctg 60

ttatagcaac aatggggac 79

<210> 32
<211> 79
<212> DNA
<213> Artificial

<220>
<223> oligonucleotide for improved targeted nucleotide exchange

<220>
<221> modified_base
<222> (46)..(47)
<223> 5-propynyl-2-deoxynucleoside

<220>
<221> modified_base
<222> (49)..(50)
<223> 5-propynyl-2-deoxynucleoside

<400> 32
caacaatagg agtttcctga aaagcatcag tacctatcat cctacgttgc acttgacctg 60

ttatagcaac aatggggac 79

<210> 33
<211> 79
<212> DNA
<213> Artificial

<220>
<223> oligonucleotide for improved targeted nucleotide exchange

<220>
<221> modified_base
<222> (47)..(47)
<223> N4-ethyl-2'-deoxycytidine

<220>
<221> modified_base
<222> (49)..(49)
<223> N4-ethyl-2'deoxycytidine

<400> 33
caacaatagg agtttcctga aaagcatcag tacctatcat cctacgttgc acttgacctg 60

ttatagcaac aatggggac 79

<210> 34
<211> 79
<212> DNA
<213> Artificial

<220>
<223> oligonucleotide for improved targeted nucleotide exchange

<220>
<221> modified_base
<222> (46)..(46)
<223> N4-ethyl-2'deoxycytidine

<220>
<221> modified_base
<222> (50)..(50)
<223> N4-ethyl-2'deoxycytidine

<400> 34
caacaatagg agtttcctga aaagcatcag tacctatcat cctacgttgc acttgacctg 60

ttatagcaac aatggggac 79

<210> 35
<211> 79
<212> DNA
<213> Artificial

<220>
<223> oligonucleotide for improved targeted nucleotide exchange

<220>
<221> modified_base
<222> (46)..(47)
<223> N4-ethyl-2'deoxycytidine

<220>
<221> modified_base
<222> (49)..(50)
<223> N4-ethyl-2'deoxycytidine

<400> 35
caacaatagg agtttcctga aaagcatcag tacctatcat cctacgttgc acttgacctg 60

ttatagcaac aatggggac 79

<210> 36
<211> 24
<212> DNA
<213> Artificial

<220>
<223> oligonucleotide for improved targeted nucleotide exchange

<400> 36
tgtgcccagt cgtagccgaa tagc 24

<210> 37
<211> 24
<212> DNA
<213> Artificial

<220>
<223> Modified oligonucleotide

<220>
<221> modified_base
<222> (1)..(1)
<223> LOCKED NUCLEIC ACID

<220>
<221> modified_base
<222> (24)..(24)
<223> LOCKED NUCLEIC ACID

<400> 37
tgtgcccagt cgtagccgaa tagc 24

<210> 38
<211> 24

<212> DNA
<213> Artificial

<220>
<223> oligonucleotide for improved targeted nucleotide exchange

<220>
<221> modified_base
<222> (1)..(2)
<223> LOCKED NUCLEIC ACID

<220>
<221> modified_base
<222> (23)..(24)
<223> LOCKED NUCLEIC ACID

<400> 38
tgtgcccagt cgtagccgaa tagc 24

<210> 39
<211> 24
<212> DNA
<213> artificial

<220>
<223> oligonucleotide for improved targeted nucleotide exchange

<220>
<221> modified_base
<222> (1)..(3)
<223> LOCKED NUCLEIC ACID

<220>
<221> modified_base
<222> (22)..(24)
<223> LOCKED NUCLEIC ACID

<400> 39
tgtgcccagt cgtagccgaa tagc 24

<210> 40
<211> 24
<212> DNA
<213> artificial

<220>
<223> oligonucleotide for improved targeted nucleotide exchange

<220>
<221> modified_base
<222> (1)..(4)
<223> LOCKED NUCLEIC ACID

<220>
<221> modified_base
<222> (21)..(24)
<223> LOCKED NUCLEIC ACID

<400> 40
tgtgcccagt cgtagccgaa tagc 24

<210> 41
<211> 24

<212> DNA
<213> artificial

<220>
<223> oligonucleotide for improved targeted nucleotide exchange

<220>
<221> modified_base
<222> (10)..(10)
<223> LOCKED NUCLEIC ACID

<220>
<221> modified_base
<222> (14)..(14)
<223> LOCKED NUCLEIC ACID

<400> 41
tgtgcccagt cgtagccgaa tagc 24

<210> 42
<211> 24
<212> DNA
<213> artificial

<220>
<223> oligonucleotide for improved targeted nucleotide exchange

<220>
<221> modified_base
<222> (9)..(9)
<223> Locked nucleic acid

<220>
<221> modified_base
<222> (15)..(15)
<223> Locked nucleic acid

<400> 42
tgtgcccagt cgtagccgaa tagc 24

<210> 43
<211> 24
<212> DNA
<213> artificial

<220>
<223> oligonucleotide for improved targeted nucleotide exchange

<220>
<221> modified_base
<222> (8)..(8)
<223> lOCKED NUCLEIC ACID

<220>
<221> modified_base
<222> (16)..(16)
<223> lOCKED NUCLEIC ACID

<400> 43
tgtgcccagt cgtagccgaa tagc 24

<210> 44
<211> 24

<212> DNA
<213> artificial

<220>
<223> oligonucleotide for improved targeted nucleotide exchange

<220>
<221> modified_base
<222> (8)..(8)
<223> LOCKED NUCLEIC ACID

<220>
<221> modified_base
<222> (10)..(10)
<223> LOCKED NUCLEIC ACID

<220>
<221> modified_base
<222> (14)..(14)
<223> LOCKED NUCLEIC ACID

<220>
<221> modified_base
<222> (16)..(16)
<223> LOCKED NUCLEIC ACID

<400> 44
tgtgcccagt cgtagccgaa tagc 24

<210> 45
<211> 24
<212> DNA
<213> artificial

<220>
<223> oligonucleotide for improved targeted nucleotide exchange

<220>
<221> modified_base
<222> (11)..(11)
<223> LOCKED NUCLEIC ACID

<220>
<221> modified_base
<222> (13)..(13)
<223> LOCKED NUCLEIC ACID

<400> 45
tgtgcccagt cgtagccgaa tagc 24

<210> 46
<211> 24
<212> DNA
<213> artificial

<220>
<223> oligonucleotide for improved targeted nucleotide exchange

<220>
<221> modified_base
<222> (11)..(11)
<223> N4-ethyl-deoxycytidine

<220>

```
<221>  modified_base
<222>  (16)..(16)
<223>  N4-ethyl-deoxycytidine

<400>  46
tgtgcccagt cgtagccgaa tagc                                          24


<210>  47
<211>  24
<212>  DNA
<213>  artificial

<220>
<223>  oligonucleotide for improved targeted nucleotide exchange


<220>
<221>  modified_base
<222>  (11)..(11)
<223>  5-propynyl-2-deoxycytidine

<220>
<221>  modified_base
<222>  (13)..(13)
<223>  5-propynyl-2-deoxyuracil

<400>  47
tgtgcccagt cgtagccgaa tagc                                          24
```